# EUROPEAN PATENT APPLICATION

(11) **EP 4 230 740 A1**
(43) Date of publication of application: **23.08.2023**
(21) Application number: 20957014.2
(22) Date of filing: 13.10.2020
(51) Int. Cl.: C12N 15/12, C12N 15/113, A61K 48/00, A61P 27/02

(54) **NUCLEIC ACID MOLECULE FOR TREATING BIETTI CRYSTALLINE DYSTROPHY, AND USE THEREOF**

(71) Applicant: Chigenovo Co., Ltd., Beijing 102206 (CN)
(72) Inventor: YANG, Liping, Beijing 100191 (CN); MENG, Xiang, Beijing 100191 (CN); CHEN, Shaohong, Beijing 102206 (CN)
(74) Representative: De Clercq & Partners
(86) International application number: PCT/CN2020/120692
(87) International publication number: WO 2022/077233

(57) **Abstract**

The present application relates to a gRNA targeting polypeptide 2, subfamily V, family 4, cytochrome P450 (CYP4V2) gene. The present application also relates to a donor nucleic acid molecule comprising a human CYP4V2 nucleotide sequence. The present application further relates to use of the gRNA and the donor nucleic acid molecule in the treatment of Bietti crystalline dystrophy.

## Description

### TECHNICAL FIELD

The application relates to the field of biomedicine, in particular to gRNAs and donor nucleic acid molecules for treating Bietti crystalline dystrophy by gene editing.

### BACKGROUND

Bietti crystalline dystrophy (BCD), also known as crystalline retinitis pigmentosa, Bietti Crystalline Comeoretinal Dystrophy, Bietti Crystalline Retinopathy, Bietti's Retinal Dystrophy, is a blinding, autosomal recessive retinal degenerative disease. The CYP4V2 gene is one of the BCD pathogenic genes discovered so far (Li et al., Am J Hum Genet. 74:817-826, 200). CYP4V2 belongs to the cytochrome P450 superfamily and is a member of heme-thiolate protein cytochrome P450 subfamily 4 (CYP4).

Currently, there are many methods for treating this disease, such as gene replacement therapy in which the CYP4V2 wild-type gene is transfected into the mutant cells using a viral transfection system or other transfection systems (such as AAV, lentivirus, retrovirus) such that the mutant cells can express the wild-type CYP4V2. This approach can only partially restore the function of mutant cells, and the efficacy is limited. The reason is that the mutant gene products still exist in the cells, and these mutant proteins will exhibit competitive inhibition with respect to normal gene products. Thus, a safer and more effective therapy is urgently needed to be discovered.

The information disclosed in this background is only intended to increase the understanding on the general background of the present application, and should not be considered as acknowledging or implying in any form that this information constitutes prior art well-known to those skilled in the art.

### SUMMARY

The present application provides a gRNA specifically targeting polypeptide 2, subfamily V, family 4, cytochrome P450 (CYP4V2), which specifically targets c.802-8_810del17bpinsGC mutation site. The gRNA has a good cleavage effect on the mutation site such that the original mutated CYP4V2 gene products are absent in the cells. The present application also provides a donor nucleic acid molecule, comprising a correct CYP4V2 nucleotide sequence without c.802-8_810del17bpinsGC mutation site. The donor nucleic acid molecule can repair the CYP4V2 mutation site in the mutant cells after the endogenous CYP4V2 is cleaved by the gRNA, to produce the CYP4V2 protein with normal functions, which exhibiting a good repair effect. The present application provides a vector comprising the gRNA and/or the donor nucleic acid molecule. It can enable CYP4V2-mutated cells to express the correct polypeptide 2, subfamily V, family 4, cytochrome P450, with a good gene editing and repair efficiency.

In one aspect, the present application provides a gRNA specifically targeting polypeptide 2, subfamily V, family 4, cytochrome P450 (CYP4V2) gene, which specifically binds to the nucleotide sequence within 200 bp upstream or downstream of c.802-8_810del17bpinsGC mutation site.

In certain embodiments, the gRNA comprises a nucleotide sequence set forth in any of SEQ ID NOs: 74, 78-80, and 82-84.

In certain embodiments, the gRNA comprises 5'-(X)n-SEQ ID NO: 74, 78-80, 82-84-skeleton sequence-3', wherein X is a base selected from any of A, U, C and G, and n is any integer from 0-15.

In certain embodiments, the gRNA is a single-stranded guide RNA (sgRNA).

In another aspect, the present application provides one or more isolated nucleic acid molecule(s) encoding the gRNA specifically targeting CYP4V2 gene.

In another aspect, the present application provides a donor nucleic acid molecule comprising a normal wild-type nucleotide sequence within 800 bp upstream or downstream of c.802-8_810del17bpinsGC mutation site of CYP4V2 gene.

In certain embodiments, the nucleic acid molecule comprises a nucleotide sequence set forth in any of SEQ ID NOs: 64-66.

In another aspect, the present application provides a vector comprising the isolated nucleic acid molecule and/or the donor nucleic acid molecule.

In certain embodiments, the isolated nucleic acid molecule and the donor nucleic acid molecule are in a same vector.

In certain embodiments, the vector is a viral vector.

In another aspect, the present application provides a cell comprising the isolated nucleic acid molecule, the donor nucleic acid molecule, and/or the vector.

In certain embodiments, the cell comprises HEK293 cells, renal epithelial cells, and/or induced pluripotent stem cells.

In certain embodiments, the cell is modified to have the differentiation capacity.

In certain embodiments, the cell can be differentiated into a 3D-retinal organoid.

In another aspect, the present application provides a pharmaceutical composition comprising the gRNA, the one or more isolated nucleic acid molecule(s), the donor nucleic acid molecule, and/or the vector, as well as a pharmaceutically acceptable carrier.

In another aspect, the present application provides use of the gRNA, the one or more isolated nucleic acid molecule(s), the donor nucleic acid molecule, and/or the vector in the manufacture of a medicament for treating a disease, wherein the disease comprises a disease caused by c.802-8_810del17bpinsGC mutation in the CYP4V2 gene.

In certain embodiments, the disease comprises Bietti crystalline dystrophy.

In another aspect, the present application provides a method for treating the Bietti crystalline dystrophy, comprising the following step: introducing the gRNA, the one or more isolated nucleic acid molecule(s), the donor nucleic acid molecule, and/or the vector into a subject in need thereof.

In certain embodiments, the introducing results in the CYP4V2 protein with normal functions.

In certain embodiments, the introducing comprises injection.

In certain embodiments, the introducing comprises injection in the subretinal space.

In another aspect, the present application provides a method for regulating the expression of CYP4V2 gene in cells, comprising introducing the gRNA, the one or more isolated nucleic acid molecule(s), and/or the vector into the cells.

Other aspects and advantages of the present application can be readily appreciated by those skilled in the art from the detailed descriptions below. Only exemplary embodiments of the present application are shown and described in the detailed descriptions below. As recognized by those skilled in the art, the disclosure of the present application will enable those skilled in the art to make changes to the specific embodiments without departing from the spirit and scope of the invention disclosed in the present application. Accordingly, the accompanying drawings and the descriptions in the specification of the present application are only exemplary and not limitative.

### DESCRIPTION OF THE DRAWINGS

The specific features of the invention disclosed in the present application are set forth in the appended claims. The features and advantages of the invention disclosed in the present application can be better understood by reference to the exemplary embodiments described in detail below and the accompanying drawings. Brief descriptions of the accompanying drawings are as follows:

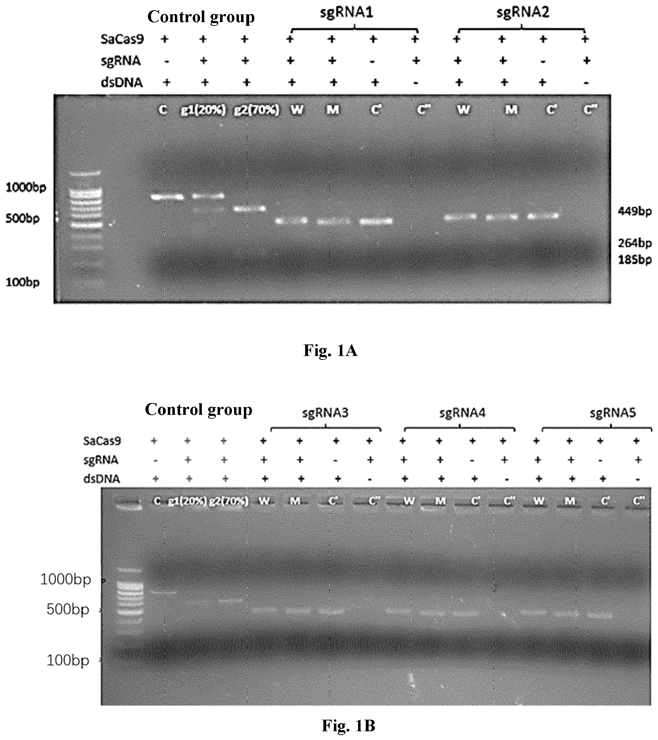

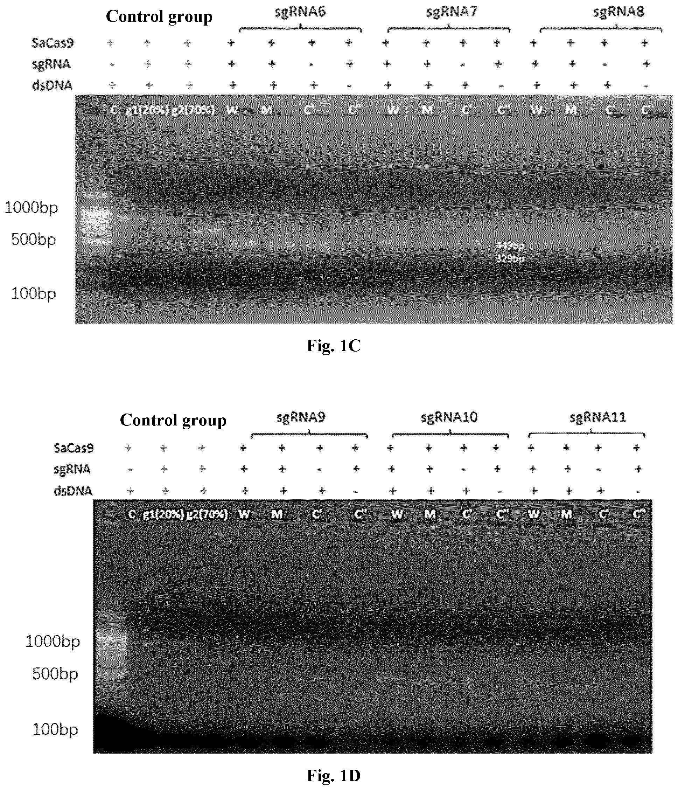

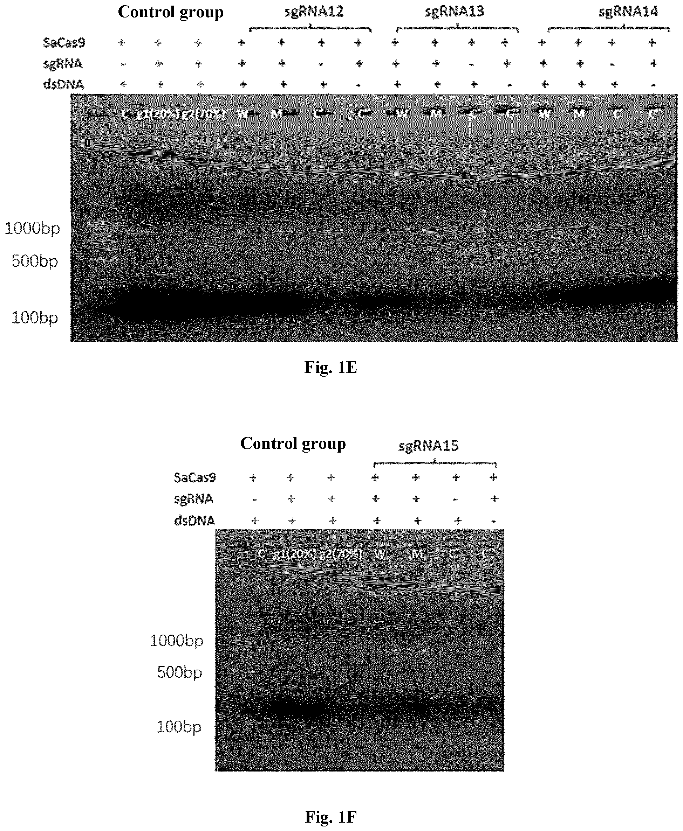

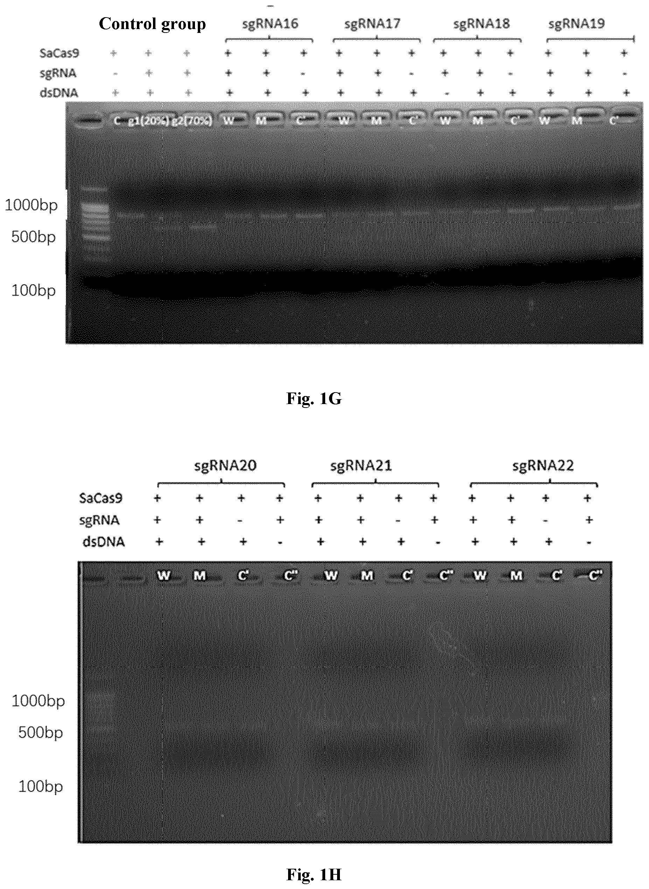

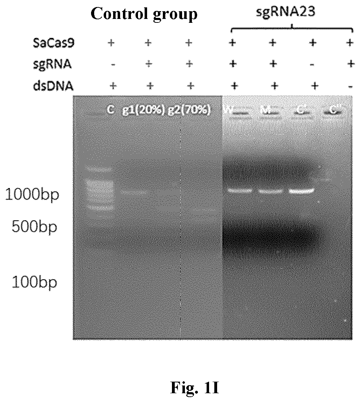

Fig. 1A shows the dsDNA splicing result of the mutated gene by sgRNA1-2 described herein; Fig. 1B shows the dsDNA splicing result of the mutated gene by sgRNA3-5 described herein; Fig. 1C shows the dsDNA splicing result of the mutated gene by sgRNA6-8 described herein; Fig. 1D shows the dsDNA splicing result of the mutated gene by sgRNA9-11 described herein; Fig. 1E shows the dsDNA splicing result of the mutated gene by sgRNA12-14 described herein; Fig. 1F shows the dsDNA splicing result of the mutated gene by sgRNA15 described herein; Fig. 1G shows the dsDNA splicing result of the mutated gene by sgRNA16-19 described herein; Fig. 1H shows the dsDNA splicing result of the mutated gene by sgRNA20-22 described herein; Fig. 1I shows the dsDNA splicing result of the mutated gene by sgRNA23 described herein.
Fig. 2 shows the plasmid map of the AAV-saCas9-puro vector described herein.
Fig. 3 shows the results of T7E1 digestion experiments on cleaved fragments by sgRNA8, sgRNA12, sgRNA13, sgRNA14, sgRNA16, sgRNA17, and sgRNA18.
Fig. 4A shows the gene sequencing result of the PCR fragment containing the cleavage site of the genomic DNA after sgRNA13 cleavage, Fig. 4B shows the gene sequencing result of the PCR fragment containing the cleavage site of the genomic DNA after sgRNA17 cleavage, and Fig. 4C shows the gene sequencing result of the PCR fragment containing the cleavage site of the genomic DNA after sgRNA18 cleavage.
Fig. 5 shows a bar graph of the cleavage efficiencies of sgRNA13, sgRNA17, sgRNA18, sgRNA13+17, and sgRNA13+18 in 293T cells.
Fig. 6 shows the design of the donor sequence.

### DESCRIPTION OF EMBODIMENTS

The embodiments of the invention in the present application are described below by certain specific examples, and those skilled in the art can easily understand other advantages and effects of the invention in the present application from the disclosure of this specification.

### Definitions of terms

As used herein, the term "CYP4V2" generally refers to a protein that is member 2 of subfamily V of cytochrome P450 family 4. The term "cytochrome P450," also known as CYP450, usually refers to a family of ferroheme proteins, belonging to a class of monooxygenases, and involved in the metabolism of endogenous substances or exogenous substances including drugs and environmental compounds. According to the homology degree of amino acid sequence, the members are divided into three levels: family, subfamily, and individual enzymes. The cytochrome P450 enzyme system may be abbreviated as CYP, wherein the family is represented by Arabic number, the subfamily is represented by English capital letter, and the individual enzyme is represented by Arabic number, such as CYP4V2 herein. The human CYP4V2 gene (HGNC: 23198; NCBI ID: 285440) has a full length of 19.28kb, located at 4q35, has 11 exons, and plays an important role in fatty acid metabolism (Kumar S., Bioinformation, 2011, 7:360-365). The CYP4V2 described herein may also comprise its functional variants, fragments, homologues, and the like. CYP4V2 is expressed almost in all tissues, but is expressed at a higher level in the retina and retinal pigment epithelium while at a slightly lower level in the cornea tissues. The mutations in the CYP4V2 gene may be associated with Bietti crystalline dystrophy and/or retinitis pigmentosa.

As used herein, the term "c.802-8_810del17bpinsGC" is a relatively common CYP4V2 gene mutation, involving the removal of 17 base pairs at the 3' end of intron 6 and the 5' end of exon 7, which results in the deletion of exon 7 and the changes in the structure and activity of the transcribed protein. An exemplary sequence between intron 6 and exon 7 of wild-type CYP4V2 may be as follows:
caaacagaagcatgtgattatcattcaaaTCATACAGGTCATCGCTgaacgggccaatgaaatgaacgccaatga (SEQ ID NO: 90). An exemplary c.802-8_810dell7insGC mutant sequence may be as follows (17 bp deletion in the sequence between intron 6 and exon 7 (the sequence in capital letters in the wild-type), with the insertion of GC (in capital letters)):
caaacagaagcatgtgattatcattcaaaGCgaacgggccaatgaaatgaacgccaatga (SEQ ID NO: 91). (Xiao et al., Biochem Biophys Res Commun. 409:181-6, 2011; Meng et al., 2014, Mol. Vis., 20:1806-14; Wada et al., Am J Ophthalmol. 139:894-9, 2005; Jiao et al., European Journal of Human Genetics (2017) 25, 461-471). As used herein, "c.802-8_810del17bpinsGC mutation site" may be at the deletion portion or at the position where CG is added, in the nucleotide sequence set forth in SEQ ID NO: 91. The term "within 800 bp upstream or downstream of c.802-8_810del17bpinsGC mutation site" may comprise, in
caaacagaagcatgtgattatcattcaaaTCATACAGGTCATCGCTgaacgggccaatgaaatgaacgccaatga (SEQ ID NO: 90), the nucleotides within about 800 bp towards the 5' end direction from the base (i.e., "a") linked to the N-terminus of the nucleotide sequence indicated by capital letters, or the nucleotides within about 800 bp towards the 3' end direction from the base (i.e., "g") linked to the C-terminus of the nucleotide sequence indicated by capital letters.

As used herein, the term "mutation" generally refers to the difference(s) in the amino acid or nucleic acid sequence of a specific protein or nucleic acid (gene, RNA) relative to the wild-type protein or nucleic acid, respectively. A mutated protein or nucleic acid may be expressed by or found in one allele (heterozygous) or both alleles (homozygous) of a gene. The mutation comprises deletion mutation, truncation, inactivation, disruption, substitution mutation, or translocation. These types of mutations are well known in the art. As used herein, those involving CYP4V2 mutation include, but not limited to, missense, duplication, splice site, frameshift, deletion, insertion, insertion or indel, nonsense, polymorphism (e.g., single nucleotide polymorphism), premature termination, and whole deletion of CYP4V2 gene.

As used herein, the term "isolated nucleic acid molecule" is one which is separated from other nucleic acid molecules present in the natural source of said nucleic acid. Such an isolated nucleic acid molecule is removed or separated from its usual or natural environment, or the molecule is produced in such a way that it is not present in its usual or natural environment. It is isolated from polypeptides, peptides, lipids, carbohydrates, other polynucleotides, or other materials in the usual or natural environment. The isolated nucleic acid molecule described herein may encode RNA, for example, may encode a gRNA specifically targeting CYP4V2 gene.

As used herein, the term "donor nucleic acid molecule" generally refers to a nucleic acid molecule that provides a heterologous nucleic acid sequence to a recipient (e.g., receiving the nucleic acid molecule).

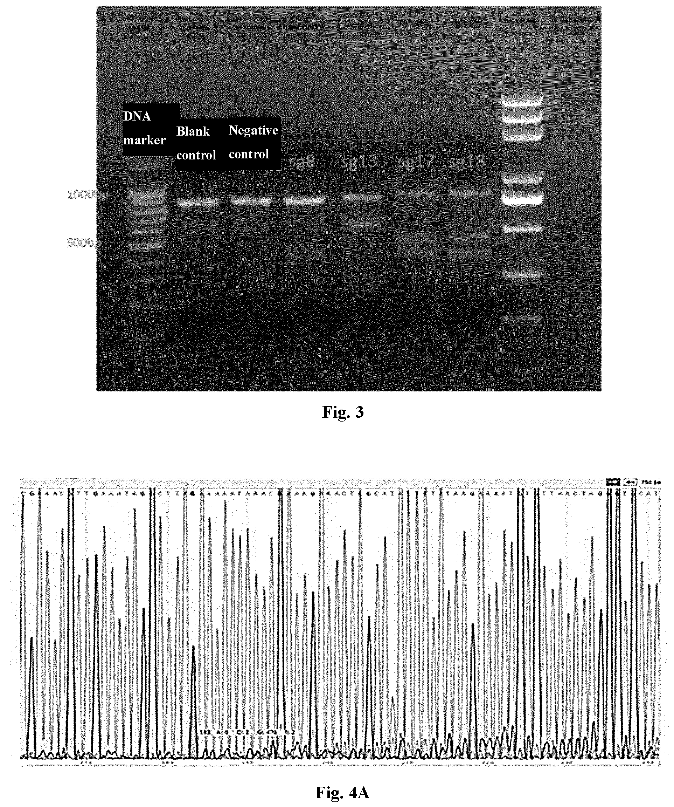

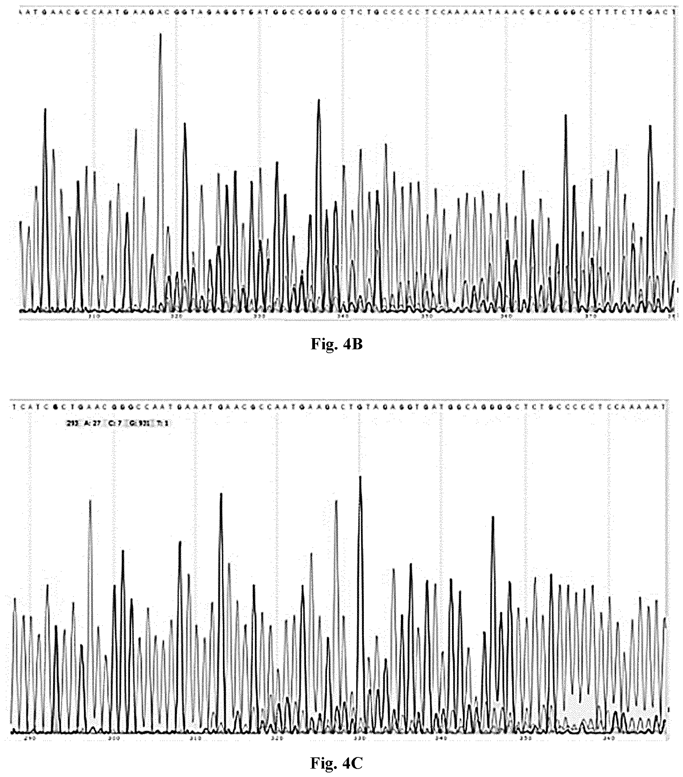

As used herein, the term "skeleton sequence" generally refers to other parts of gRNA other than those that recognize or hybridize the target sequence, and may comprise the sequence between the gRNA pairing sequence and the transcription terminator in the sgRNA. Generally, the skeleton sequence does not change with the target sequence, nor does it affect the recognition of the target sequence by the gRNA. Thus, the skeleton sequence may be any feasible sequence in the prior art. The structure of the skeleton sequence can be found in the parts other than the spacer sequences described in panels A and B of Figure 1, panels A, B, and C of Figure 3, as well as panels A, B, C, D, and E of Figure 4 in the literature Nowak et al. Nucleic Acids Research 2016. 44:9555-9564.

As used herein, the term "vector" generally refers to a nucleic acid molecule capable of self-replication in a suitable host, which can transfer the inserted nucleic acid molecule (e.g., exogenous sequence) into and/or among host cells. The vector may include vectors primarily used for insertion of DNA or RNA into cells, vectors primarily used for replication of DNA or RNA, and vectors primarily used for expression of transcription and/or translation of DNA or RNA. The vector also includes a vector having a number of the above-mentioned functions. The vector may be a polynucleotide capable of being transcribed and translated into a polypeptide when introduced into a suitable host cell. Typically, the vector can produce the desired expression product by culturing a suitable host cell containing the vector. The vector described herein may comprise, for example, expression vectors, including viral vectors (lentiviral vectors and/or retroviral vectors), phage vectors, phagemids, cosmids, artificial chromosomes such as yeast artificial chromosome (YAC), bacterial artificial chromosome (BAC) or P1-derived artificial chromosome (PAC), and/or plasmids.

As used herein, the term "gRNA" generally refers to guide RNA, a type of RNA molecule. In nature, crRNA and tracrRNA usually exist as two independent RNA molecules to form gRNA. The term "crRNA," also known as CRISPR RNA, usually refers to a nucleotide sequence complementary to the target DNA to be targeted, and the term "tracrRNA" usually refers to a scaffold RNA capable of binding to the Cas nuclease. The crRNA and tracRNA can also be fused into a single strand. At this time, the gRNA can also be called as single guide RNA (sgRNA). The sgRNA has become the most common form of gRNA used by those skilled in the art in CRISPR technology. Therefore, the terms "sgRNA" and "gRNA" herein may have a same meaning. The sgRNA can be artificially synthesized, or can also be prepared from a DNA template *in vitro* or *in vivo.* The sgRNA can bind to the Cas nuclease or can also target the DNA of interest, which can guide the Cas nuclease to cleave the DNA site complementary to the gRNA.

As used herein, the term "specifically targeting" generally refers to the interaction between two molecules (e.g., molecule A and molecule B) (e.g., molecule A specifically recognizes and/or binds to molecule B (e.g., target)). The molecule A interacts with molecule B to a statistically significant degree compared to interactions with the molecules other than molecule B. The interaction may be covalent or non-covalent. When referring to a polynucleotide, the term "specifically targeting" may refer to the complementary base pairing relationship of molecule A (or a strand thereof) with molecule B (or a strand thereof). As used herein, the term "specifically targeting" may refer to the process in which gRNA recognizes and/or binds to a target sequence.

As used herein, the terms "target nucleic acid," "nucleic acid of interest," "target sequence," and "target region" are used interchangeably, and usually refer to a nucleic acid sequence that can be recognized by a gRNA. The target nucleic acid may refer to a double-stranded nucleic acid or a single-stranded nucleic acid.

As used herein, the term "3D-retinal organoid" generally refers to an artificially grown retina having a three-dimensional structure, capable of self-renewal and self-organization, and exhibiting basic retinal functions (e.g., light perception). The 3D-retinal organoid can be differentiated from primary tissues or stem cells (e.g., pluripotent stem cells), with all the cells in the retina required for receiving lights and sending signals to the brain.

As used herein, the term "Bietti crystalline dystrophy" generally refers to a class of autosomal recessive ocular diseases. The symptoms mainly include crystals (transparent coverings) in the cornea; small, yellow or white, crystalline deposits deposited in the photosensitive tissues of the retina; and progressive atrophy of the retina, choriocapillary, and choroid. The Bietti crystalline dystrophy may include a disease caused by CYP4V2 gene mutation.

As used herein, the term "injection in the subretinal space" generally refers to the introduction of the substance to be introduced between the photoreceptor cells and the retinal pigment epithelium (RPE) layer. During the injection in the subretinal space, the injected material (e.g., the gRNA described herein, the one or more isolated nucleic acid molecule(s) described herein, the donor nucleic acid molecule described herein, the vector described herein, as well as a pharmaceutically acceptable carrier) creates a space between there.

As used herein, the term "cell" refers to the meaning as generally recognized in the art. This term is used in its ordinary biological sense, and does not refer to a whole multicellular organism, such as human in particular. The cell may be present in organisms such as birds, plants, and mammals, such as humans, cows, sheep, apes, monkeys, pigs, dogs, and cats. The cell may be prokaryotic (e.g., bacterial cell) or eukaryotic (e.g., mammalian or plant cell). The cell may be of somatic or germline origin, totipotent or pluripotent, divided or non-divided. The cell may also be derived from or may comprise gametes or embryos, stem cells, or fully differentiated cells.

As used herein, the term "induced pluripotent stem cell" generally refers to a somatic cell reverted to a cell in the state of totipotency under certain conditions. The totipotency refers to the ability to differentiate into all types of cells in the body and form a complete embryo or further develop into a new individual. For example, as used herein, the induced pluripotent stem cell comprises cells obtained after culture of renal epithelial cells and capable of differentiating into retinal cells.

As used herein, the term "HEK293 cell" usually refers to "human embryonic kidney cell 293", which is a cell line derived from human embryonic kidney cells. It has the characteristics of easy culture and high transfection efficiency, and is a very commonly used cell line for studying exogenous genes in the art. As used herein, the term "293T cell" or "HEK293T cell" is a kind of cell derived from "HEK293 cell", which is also a commonly used cell line in the art.

As used herein, the term "renal epithelial cell" generally refers to the epithelial cell of the kidney as collected in human urine. As used herein, it may be a source for induced pluripotent stem cells. In the art, the use of renal epithelial cells in urine to induce pluripotent stem cells is cost-effective, versatile, and suitable for all ages, genders, and races. This technique makes obtaining large amounts of patient samples much easier and less expensive than other existing manners.

As used herein, the term "pharmaceutical composition" generally refers to a composition suitable for administration to a subject in need thereof. For example, the pharmaceutical composition described herein may comprise the gRNA described herein, the one or more isolated nucleic acid molecule(s) described herein, the donor nucleic acid molecule described herein, and/or the vector described herein, as well as a pharmaceutically acceptable carrier. The term "subject" or "individual" or "animal" or "patient" is used interchangeably herein and refers to a subject (such as mammalian subject) in need of administration of the pharmaceutical composition herein. The animal subject comprises humans, non-human primates, dogs, cats, guinea pigs, rabbits, rats, mice, horses, cattle, cows, and the like, such as mice. In certain embodiments, the pharmaceutical composition may comprise a composition for subretinal, parenteral, transdermal, intracavity, intraarterial, intrathecal, and/or intranasal administration or direct injection into tissue. For example, the pharmaceutical composition is administrated to a subject by injection in the subretinal space.

In addition to the specific proteins and nucleotides mentioned herein, the present application may also encompass the use of variants, derivatives, analogs, homologues, and fragments thereof.

In the context of this application, a variant of any given sequence refers to a sequence in which a particular sequence of residues (whether amino acid or nucleotide residues) has been modified such that the polypeptide or polynucleotide substantially retains at least one endogenous functions. Variant sequences can be obtained by addition, deletion, substitution, modification, replacement, and/or variation of at least one amino acid residues and/or nucleotide residues present in a naturally occurring protein and/or polynucleotide.

As used herein, the term "derivative" generally refers to the polypeptide or polynucleotide of the present application including any substitution, variation, modification, replacement, deletion, and/or addition of one amino acid residue (or multiple amino acid residues) of the sequence, as long as the resulting polypeptide or polynucleotide substantially retains at least one endogenous function.

As used herein, the term "analog" generally refers to a polypeptide or polynucleotide that includes any mimetic of the polypeptide or polynucleotide, i.e., a chemical compound possessing at least one endogenous function of the polypeptide or polynucleotide which the mimetic mimics.

Generally, the amino acid substitutions, such as at least 1 (e.g., 1, 2, 3, 4, 5, 6, 7, 8, 9, 10, 20, or more) amino acid substitutions, can be made, as long as the modified sequence substantially retains the desired activity or ability. The amino acid substitutions can include the use of non-naturally occurring analogs.

The proteins or polypeptides used herein may also have deletions, insertions, or substitutions of amino acid residues that produce silent changes and result in functionally equivalent proteins. The deliberate amino acid substitutions can be made based on the similarity in polarity, charge, solubility, hydrophobicity, hydrophilicity, and/or amphiphilic nature of the residues, as long as the endogenous function is retained. For example, negatively charged amino acids include aspartic acid and glutamic acid; positively charged amino acids include lysine and arginine; and amino acids containing uncharged polar headgroups with similar hydrophilicity values include asparagine, glutamine, serine, threonine, and tyrosine.

As used herein, the term "homologue" generally refers to an amino acid sequence or nucleotide sequence having a certain homology to a wild-type amino acid sequence and a wild-type nucleotide sequence. The term "homology" can be equivalent to the "identity." A homologous sequence can include an amino acid sequence that may be at least 70%, 75%, 80%, 85%, 90%, 99.1%, 99.2%, 99.3%, 99.4%, 99.5%, 99.6%, 99.7%, 99.8%, or 99.9% identical to the subject sequence. Typically, a homologue will contain the same active site as the subject amino acid sequence and the like. The homology can be considered in terms of similarity (i.e., amino acid residues with similar chemical properties/functions), or it can be expressed in terms of sequence identity. As used herein, a sequence having a percent identity to any of SEQ ID NOs of an amino acid sequence or a nucleotide sequence as mentioned refers to a sequence having said percent identity over the entire length of SEQ ID NO as mentioned.

To determine the sequence identity, the sequence alignment can be performed, which can be performed by various means known to those skilled in the art, for example using BLAST, BLAST-2, ALIGN, NEEDLE, or Megalign (DNASTAR) software, and the like. Those skilled in the art can determine the appropriate parameters for alignment, including any algorithm required to achieve optimal alignment among the full-length sequences to be compared.

In the present application, the term "and/or" should be understood to mean either or both of the options.

As used herein, the term "comprise" or "include" generally means the inclusion of expressly specified features, but without the exclusion of other elements.

As used herein, the term "about" generally refers to variations above or below the specified value within the range of 0.5%-10%, such as variations above or below the specified value within the range of 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10%.

### DETAILED DESCRIPTION OF THE INVENTION

### gRNA

In one aspect, the present application provides a gRNA specifically targeting polypeptide 2, subfamily V, family 4, cytochrome P450 (CYP4V2) gene, which specifically targets the mutation site of the CYP4V2 gene.

In certain instances, one or more introns and exons of CYP4V2 may possess the removal or addition of varying pairs (e.g., 1 pair, 2 pairs, 3 pairs, 4 pairs, 10 pairs, 20 pairs, 50 pairs, 100 pairs) of bases as well as the mutation or substitution of nucleotides, leading to the structure change and function loss of the CYP4V2 protein. For example, the mutation site of the CYP4V2 gene may include the mutation sites shown in Table 1:

**Table 1. Mutation sites of CYP4V2 gene**

| Exon or intron | Mutation site | Mutation result |
|---|---|---|
| E1 | c.31C>T | p.QHX |
| | c.64C>G | p.L22V |
| | c.71T>C | p.L24P |
| | c.77G>A | p.G26D |
| | c.130T>A | p.W44R |
| | c.134A>C | p.Q45P |
| | c.181G>A | p.G61S |
| | c.197T>G | p.M66R |
| IVS1 | c.214+lG>A | Exon 1 deletion |
| | c.214+25delT | Not available |
| | c.215-2A>G | Exon 2 deletion |
| | c.215-lG>A | Exon 2 deletion |
| E2 | c.219T>A | p.F73L |
| | c.237G>T | p.E79D |
| | c.253C>T | p.R85C |
| | c.277T>C | p.W93R |
| | c.283G>A | p.G95R |
| | c.327G>A | Not available |
| IVS2 | c.327+lG>A | p.E72Gfs*5 |
| | c.327+HG>C | Not available |
| E3 | c.332T>C | p.ΠIIT |
| | c.335T>G | p.L112* |
| | c.367A>G | P.M123V |
| | c.400G>T | p.G134* |
| | c.413+2T>G | Splice variation |
| | c.283G>A | p.G95R |
| E4 | c.518T>G | p.L173W |
| E5 | c.637_641delAGTAA | p. S213* |
| | c.655T>C | p.Y219H |
| E7 | c.802-8_806dell3 | Exon 7 deletion |
| | c.802-8_810del17insGC | Exon 7 deletion |
| | c.802-8_810del17bpinsGT | Splice variation |
| | c.810delT | p.(Glu271Argfs*34) |
| | c.838G>T | p.E280* |
| | c.958C>T | p.R320* |
| | c.971A>T | p.D324V |
| | c.974C>T | p.T325I |
| | c.965_7delAAG | p.321delE |
| IVS7 | c.985+3A>G | Not available |
| E8 | c.992A>C | p.H331P |
| | c.998C>A | p.T333K |
| | c.1020G>A | p.W340* |
| | c.1021T>C | p.S341P |
| | c.1027T>G | p.Y343D |
| | c.lO62dupA | p.V355Sfs*4 |
| | c.992A>G | p.H331P |
| | c.994G>A | p.D332N |
| | c.1062insA | p.V355Sfs*3 |
| | c.1062-1063insA | p.V355Sfs*2 |
| IVS8 | c.1091-2A>G | Exon 9 deletion |
| E9 | c.H57A>C | p.K386T |
| | c.H68C>T | p.R390C |
| | c.H69G>A | p.R390H |
| | c.H78C>T | p.P393L |
| | c.H87C>T | p.P396L |
| | c.H98C>T | p.R400C |
| | c.H99G>A | p.R400H |
| | c.1219G>T | p.E407* |
| | c.1225+1 G>A | p.(G364_V408del) |
| | c. 1091-2A>G | Splice variation |
| E10 | c.1226-6_1235dell6 | Exon 10 deletion |
| | c.1328G>A | p.R443Q |
| | c.1348C>T | p.Q450* |
| | c.1355G>A | p.R452H |
| | c. 13 72G>A | p.V458M |
| | c.1393A>G | p.R465G |
| | c.1396A>G | p.N466D |
| | c.1399T>C | p.C467R |
| | c.1441delT | p.(Ser481Argfs*4) |
| | c.1445C>T | p.S482* |
| E11 | c.1523G>A | p.R508H |
| | c.1526C>T | p.P509L |
| | c.604G>A | p.(Glu202Lys) |
| | c.242C>G | p.(Thr81Arg) |
| | c.604+4A>G | p.(?) |
| | c.1249dup | p.(Thr417Asnfs*2) |
| | Whole CYP4V2 deletion | * |
| | c.1544T>G | p.I515S |

In certain instances, the mutation site of the CYP4V2 gene comprises c.802-8_810del17bpinsGC. In the present application, the term "c.802-8_810del17bpinsGC" is a relatively common CYP4V2 gene mutation, involving the removal of 17 pairs of bases at the 3' end of intron 6 and the 5' end of exon 7, which results in the deletion of exon 7 and the changes in the structure and activity of the transcribed protein. In certain instances, c.802-8_810del17bpinsGC may also incorporate other types of mutations, such as c.992A>C(p.H331P) and c. 1091-2A>G (Exon 9del).

In certain instances, the gRNA may specifically bind to a nucleotide sequence 100 bp, 120 bp, 140 bp, 160 bp, 180 bp, 200 bp, 250 bp, 300 bp, 350 bp, 400 bp, or 500 bp upstream or downstream of c.802-8_810del17bpinsGC mutation site. In certain instances, the gRNA may specifically bind to a nucleotide sequence 200 bp upstream or downstream of c.802-8_810del17bpinsGC mutation site. In certain instances, the gRNA may specifically bind to a nucleotide sequence having at least 70% (for example, at least 75%, at least 80%, at least 85%, at least 90%, at least 91%, at least 92%, at least 93%, at least 94%, at least 95%, at least 96%, at least 97%, at least 98%, at least 99%, or at least 100%) sequence identity to the nucleotide sequence 200 bp upstream or downstream of c.802-8_810del17bpinsGC mutation site.

In certain instances, the gRNA may specifically bind to a nucleotide sequence complementary to the nucleotide sequence 100 bp, 120 bp, 140 bp, 160 bp, 180 bp, 200 bp, 250 bp, 300 bp, 350 bp, 400 bp, or 500 bp upstream or downstream of c.802-8_810del17bpinsGC mutation site. In certain instances, the gRNA may specifically bind to a nucleotide sequence complementary to the sequence 200 bp upstream or downstream of c.802-8_810del17bpinsGC mutation site. In certain instances, the gRNA may specifically bind to a nucleotide sequence complementary to the nucleotide sequence having at least 70% (for example, at least 75%, at least 80%, at least 85%, at least 90%, at least 91%, at least 92%, at least 93%, at least 94%, at least 95%, at least 96%, at least 97%, at least 98%, at least 99%, or at least 100%) sequence identity to the amino acid sequence complementary to the sequence 200 bp upstream or downstream of c.802-8_810del17bpinsGC mutation site.

The gRNA described herein may bind to the sequence in the target nucleic acid (e.g., mutation site of CYP4V2 gene). The gRNA can interact with the target nucleic acid in a sequence-specific manner by hybridization (i.e., base pairing). The nucleotide sequence of the gRNA may vary depending on the sequence of the target nucleic acid of interest.

In the present application, the gRNA may comprise a nucleotide sequence set forth in any of SEQ ID NO: 74, SEQ ID NO: 78, SEQ ID NO: 79, SEQ ID NO: 80, SEQ ID NO: 82, SEQ ID NO: 83, and SEQ ID NO: 84. In the present application, the gRNA may comprise a nucleotide sequence having at least 70% (for example, at least 75%, at least 80%, at least 85%, at least 90%, at least 91%, at least 92%, at least 93%, at least 94%, at least 95%, at least 96%, at least 97%, at least 98%, at least 99%, or at least 100%) sequence identity to the nucleotide sequence set forth in any of SEQ ID NO: 74, SEQ ID NO: 78, SEQ ID NO: 79, SEQ ID NO: 80, SEQ ID NO: 82, SEQ ID NO: 83, and SEQ ID NO: 84.

In the present application, the gRNA may comprise a nucleotide sequence set forth in any of SEQ ID NO: 74, SEQ ID NO: 79, SEQ ID NO: 83, and SEQ ID NO: 84. In the present application, the gRNA may comprise a nucleotide sequence having at least 70% (for example, at least 75%, at least 80%, at least 85%, at least 90%, at least 91%, at least 92%, at least 93%, at least 94%, at least 95%, at least 96%, at least 97%, at least 98%, at least 99%, or at least 100%) sequence identity to the nucleotide sequence set forth in any of SEQ ID NO: 74, SEQ ID NO: 79, SEQ ID NO: 83, and SEQ ID NO: 84.

In the present application, the gRNA may comprise a nucleotide sequence set forth in any of SEQ ID NO: 79, SEQ ID NO: 83, and SEQ ID NO: 84. In the present application, the gRNA may comprise a nucleotide sequence having at least 70% (for example, at least 75%, at least 80%, at least 85%, at least 90%, at least 91%, at least 92%, at least 93%, at least 94%, at least 95%, at least 96%, at least 97%, at least 98%, at least 99%, or at least 100%) sequence identity to the nucleotide sequence set forth in any of SEQ ID NO: 79, SEQ ID NO: 83, and SEQ ID NO: 84.

In the present application, the gRNA may comprise (X)n, a nucleotide sequence set forth in any of SEQ ID NOs: 74, 78-80, and 82-84, and a skeleton sequence, wherein X is a base selected from any of A, U, C and G, and n is any integer from 0-15. In the present application, the gRNA may comprise 5'-(X)n-nucleotide sequence set forth in any of SEQ ID NOs: 74, 78-80, and 82-84-skeleton sequence-3', wherein X is a base selected from any of A, U, C and G, and n is any integer from 0-15. For example, the skeleton sequence described herein may comprise those from AAV-saCas9-puro.

In certain instances, the gRNA may be a single-stranded guide RNA (sgRNA).

The present application also provides a sgRNA combination, and the sgRNA combination may comprise the sgRNA described herein. For example, the sgRNA may comprise two or more sgRNAs disclosed herein. For example, the sgRNA combination may comprise sgRNA13 and sgRNA17; for another example, the sgRNA combination may comprise sgRNA13 and sgRNA18; for another example, the sgRNA combination may comprise sgRNA17 and sgRNA18; for another example, the sgRNA combination may comprise sgRNA13, sgRNA17, and sgRNA18.

The present application provides one or more isolated nucleic acid molecule(s) that can encode the gRNA specifically targeting the CYP4V2 mutated gene described above. For example, the isolated nucleic acid molecule may comprise a nucleotide sequence set forth in any of SEQ ID NO: 8, SEQ ID NO: 12, SEQ ID NO: 13, SEQ ID NO: 14, SEQ ID NO: 16, SEQ ID NO: 17, and SEQ ID NO: 18.

In the present application, the gRNA sequence may be designed to hybridize to a target nucleic acid in the vicinity of a PAM sequence recognizable by the Cas nuclease. The gRNA may be completely complementary to the target sequence, or may be incompletely complementary to the target sequence. The degree of complementarity between the gRNA and its corresponding target sequence is at least 50% (e.g., at least about 55%, about 60%, about 65%, about 70%, about 75%, about 80%, about 85%, about 90%, about 95%, about 98%, or more). The "Cas nuclease" generally refers to the ability to use a CRISPR sequence (e.g., gRNA) as a guide to recognize and cleave a specific DNA strand, for example, Cas9 nuclease, Csn1 or Csx12. The Cas9 nuclease typically includes a RuvC nuclease domain and an HNH nuclease domain, which cleave two different strands of a double-stranded DNA molecule, respectively. The Cas9 nuclease has been described in different bacterial species such as *S. thermophiles, Listeria innocua* (Gasiunas, Barrangou *et al.* 2012; Jinek, Chylinski *et al.* 2012), and *S. Pyogenes* (Deltcheva, Chylinski *et al.* 2011). For example, the amino acid sequence for *Streptococcus pyogenes* Cas9 protein can be found in SwissProt database with accession number Q99ZW2; the amino acid sequence for *Neisseria meningitides* Cas9 protein can be found in UniProt database with number A1IQ68; the amino acid sequence for *Streptococcus thermophilus* Cas9 protein can be found in UniProt database with number Q03LF7; and the amino acid sequence for *Staphylococcus aureus* Cas9 protein (e.g., SaCas in the vector described herein) can be found in UniProt database with number J7RUA5. The Cas nuclease may usually recognize a specific PAM sequence in the DNA. The "PAM" refers to a protospacer adjacent motif, which is recognized as a cleavage site by a CRISPR nuclease, and the PAM varies with the nuclease (e.g., Cas9, Cpfl, etc.). The protospacer element sequence is usually located directly upstream of the PAM site. For example, the PAM may comprise a nucleotide sequence set forth in any of SEQ ID NOs: 24-38.

The gRNA and/or isolated nucleic acid molecule described herein may be delivered using a vector. In the present application, the vector (such as pX601) may or may not contain a nucleic acid encoding the Cas nuclease. In the present application, the Cas nuclease may be delivered individually as one or more polypeptides. Alternatively, the nucleic acid molecule encoding the Cas nuclease as well as one or more guide RNAs, or one or more crRNAs and tracrRNAs are delivered individually or pre-complexed together for delivery. For example, the nucleic acid molecule of the present application (e.g., the isolated nucleic acid molecule encoding the sgRNA specifically targeting the CYP4V2 gene) and the nucleic acid molecule encoding the Cas9 nuclease may be located in a same vector (e.g., a plasmid). The vector may include viral or non-viral vectors known in the art.

The non-viral delivery vector may include, but not limited to, nanoparticles, liposomes, ribonucleoproteins, positively charged peptides, small-molecule RNA conjugates, aptamer-RNA chimeras, and RNA fusion protein complexes.

In the present application, the isolated nucleic acid molecule and/or the nucleic acid molecule encoding a DNA endonuclease may be delivered via a plasmid.

In certain instances, the vector may be a viral vector, e.g., AAV, lentivirus, retrovirus, adenovirus, herpes virus, and hepatitis virus. The methods for producing the viral vector comprising the nucleic acid molecule (e.g., isolated nucleic acid molecule described herein) as part of the vector genome are well known in the art and may be performed by those skilled in the art without undue experimentation. In other instances, the vector may be a recombinant AAV virion that packages the nucleic acid molecule described herein. The methods for producing a recombinant AAV may comprise introducing the nucleic acid molecule described herein into a packaging cell line, introducing a packaging plasmid expressing the AAV rep and cap genes into the cell line, and collecting the recombinant AAV from the supernatant of the packaging cell line. Various types of cells may be used as packaging cell lines. For example, the packaging cell lines that can be used include, but are not limited to, HEK 293 cells, HeLa cells, and Vero cells.

In certain instances, the vector may be an adeno-associated virus (AAV) vector. In the present application, the term "adeno-associated virus vector" generally refers to the vectors derived from naturally occurring and available adeno-associated viruses as well as artificial AAVs. The AAV may include different serotypes, such as AAV1, AAV2, AAV3, AAV4, AAV5, AAV6, AAV7, AAV8, AAV9, AAV10, AAV1 1, AAV12, or AAV13, as well as any AAV variant or mixture. The AAV genome usually has inverted terminal repeats (ITRs) at both ends. The term "ITR" or "inverted terminal repeats" refers to a stretch of nucleic acid sequence present in the AAV and/or recombinant AAV, which can be form a T-shaped palindrome required for the completion of AAV lysis and latency lifecycle. The techniques for producing the AAV vector are standard techniques in the art, and include providing to cells the polynucleotide to be delivered, the rep and cap genes, and the AAV genome to be packaged for helper virus function. The production of AAV vector typically requires the presence of the following components within a single cell (herein referred to as packaging cell): rAAV genome, AAV rep and cap genes separate from (e.g., not in) the rAAV genome, as well as helper virus. The AAV rep and cap genes may be from any AAV serotype, and may also be from an AAV serotype different from the ITR of AAV genome, including but not limited to the AAV serotypes described herein.

### Donor nucleic acid molecule and vector

In another aspect, the present application also provides a donor nucleic acid molecule. In the present application, the term "donor nucleic acid molecule" generally refers to a nucleic acid molecule that provides a heterologous nucleic acid sequence to a recipient (e.g., receiving the nucleic acid molecule). In certain instances, the donor nucleic acid molecule is introduced into a recipient cell, and the DNA fragments (e.g., double-stranded DNA after breakage) that have been cleaved by the isolated nucleic acid molecule may be repaired. In some other instances, the DNA breakage may be repaired by the donor nucleic acid molecule. The repairing manner includes, but not limited to, homologous recombination (HR) repair dependent upon DNA homology and non-homologous end joining (NHEJ) repair.

In certain instances, an exogenous polynucleotide sequence may be inserted into the target nucleic acid cleavage site by HDR. The exogenous polynucleotide sequence may be referred to as a donor polynucleotide (or donor, or donor sequence, or polynucleotide donor template). The donor polynucleotide, a portion of the donor polynucleotide, a copy of the donor polynucleotide, or a portion of a copy of the donor polynucleotide may be inserted into the target nucleic acid cleavage site. The donor polynucleotide may be an exogenous polynucleotide sequence, that is, a sequence that is not naturally present at the target nucleic acid cleavage site. HDR uses the homologous sequence or donor sequence (e.g., donor nucleic acid molecule) as a template to insert a specific DNA sequence at the breakpoint. The homologous sequence may be in the endogenous genome, such as sister chromatid. Alternatively, the donor may be an exogenous nucleic acid, such as plasmid, single-stranded oligonucleotide, double-stranded oligonucleotide, or virus. These exogenous nucleic acids may comprise regions of high homology to the DNA nuclease-cleavable locus, and may also comprise additional sequences or sequence changes (including deletions that can be incorporated into the cleavable target locus). For example, the present application utilizes the homologous arm, and the exogenous gene fragment (e.g., the donor nucleic acid molecule described herein) linked to the homologous arm may be integrated into the target site (e.g., the gRNA targeting site described herein). The "homologous arm" described herein generally refers to a stretch of nucleotide sequence having homology with the nucleotide sequence at the DNA breakpoint.

Using an exogenous donor template, additional nucleic acid sequences (e.g., the targeting vector) or modifications (e.g., single or multiple base changes or deletions) may be introduced between homologous flanking regions, such that the additional or changed nucleic acid sequences are incorporated into the loci of interest. The exogenous donor may be delivered by a plasmid vector, for example, AAV vector and/or TA cloning vector (e.g., ZT4 vector).

The donor nucleic acid molecule described herein may be a nucleotide sequence, gene fragment, or homologue of the wild-type human CYP4V2. For example, the donor nucleic acid molecule comprises a nucleotide sequence within 500 bp, 700 bp, 750 bp, 800 bp, 850 bp, 900 bp, 1000 bp, 2000 bp, 3000 bp, or 5000 bp upstream or downstream of c.802-8_810del17bpinsGC mutation site. In certain instances, the donor nucleic acid may comprise 800 bp upstream or downstream of c.802-8_810del17bpinsGC mutation site. In certain instances, the donor nucleic acid may comprise a nucleotide sequence having at least 50% (for example, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or at least 100%) sequence identity to 800 bp upstream or downstream of c.802-8_810del17bpinsGC mutation site. The "nucleic acid molecule" described herein that may comprise a CYP4V2 nucleotide sequence is different from the gRNA specifically targeting CYP4V2 or the "isolated nucleic acid molecule" described herein.

For example, in the present application, the donor nucleic acid molecule may comprise a nucleotide sequence set forth in any of SEQ ID NOs: 64-66. For another example, the donor nucleic acid molecule may comprise a nucleotide sequence having at least 50% (for example, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or at least 100%) sequence identity to the nucleotide sequence set forth in any of SEQ ID NOs: 64-66.

In another aspect, the present application provides a vector, comprising the isolated nucleic acid molecule (e.g., an isolated nucleic acid molecule encoding the sgRNA specifically targeting the CYP4V2 gene) and/or the donor nucleic acid molecule (e.g., a nucleotide molecule encoding the human CYP4V2 gene).

In certain instances, the isolated nucleic acid molecule and the donor nucleic acid molecule may be located in different vectors. In some other instances, the isolated nucleic acid molecule and the donor nucleic acid molecule may be located in a same vector. In the present application, after the vector containing the isolated nucleic acid molecule and the vector containing the donor nucleic acid molecule are simultaneously introduced into a cell, the Cas nuclease may cleave the donor nucleic acid molecule and the genomic DNA of the cell, and integrate the donor nucleic acid molecule into the precise site (e.g., CYP4V2 gene fragment) in the cell genome.

In the present application, the vector may comprise one or more sgRNAs. For example, 1, 2, 3, 4, 5, 6, 7, or more than 7 sgRNAs described herein may be comprised. In certain instances, when multiple sgRNAs are used, the multiple sgRNAs may be present in one or more vectors, and may also be administrated simultaneously or sequentially. For example, the sgRNAs may be administrated in different vectors respectively, or may be administrated in a same vector. For example, the sgRNAs may be administrated in a same AAV vector. For example, the sgRNAs may be located in different vectors and administrated simultaneously. For example, the sgRNAs may be located in a same vector and administrated sequentially.

In certain embodiments, the vector is a viral vector; for example, AAV, lentivirus, retrovirus, adenovirus, herpes virus, and hepatitis virus. The methods for producing the viral vector comprising the nucleic acid molecule (e.g., isolated nucleic acid molecule described herein) as part of the vector genome are well known in the art and may be performed by those skilled in the art without undue experimentation.

### Cell, pharmaceutical composition, use, and method

The present application provides a cell, which may comprise the isolated nucleic acid molecule and/or the donor nucleic acid molecule. The cell described herein may express sgRNA and Cas nuclease, and have a good DNA cleavage effect. The cell described herein may also express the CYP4V2 protein with normal functions. The cell may include mammalian cells, e.g., cells from humans. For example, the cell may include COS cells, COS-1 cells, Chinese Hamster Ovary (CHO) cells, HeLa cells, HEK293 cells, NSO cells or myeloma cells, stem cells (e.g., pluripotent stem cells and/or totipotent stem cells), and/or epithelial cells (e.g., kidney epithelial cells and/or retinal epithelial cells). In the present application, the cell may include HEK293T cells and/or urine kidney epithelial cells. In the present application, the cells may be modified to have a differentiation potential. The differentiation potential may include the potential to differentiate into any cell type in the body: neurons, astrocytes, oligodendrocytes, retinal epithelial cells, epidermis, hair and keratinocytes, hepatocytes, pancreatic beta cells, intestinal epithelial cells, alveolar cells, hematopoietic cells, endothelial cells, cardiomyocytes, smooth muscle cells, skeletal muscle cells, kidney cells, adipocytes, chondrocytes, and/or osteocytes. For example, the cell may be reprogrammed into induced pluripotent stem cells (iPSCs) with key reprogramming genes (e.g., OCT4, KLF4, SOX2, cMYC, NANOG, and/or LIN28) overexpressed.

The cell described herein may be used to evaluate the effectiveness and safety of a substance required for gene editing therapy (e.g., sgRNA and donor nucleic acid molecule). The present application also provides a tissue model that may comprise the correct human CYP4V2 cDNA 3D-retinal organoid. The cell and the tissue model described herein may be used to evaluate the effectiveness and safety of a substance required for gene editing therapy (e.g., sgRNA and donor nucleic acid molecule). For example, after introducing the polynucleotide, the donor nucleic acid molecule, and/or vector encoding the gRNA into the cell and/or the tissue model, the expression of the gRNA and the correct CYP4V2 protein can be detected (e.g., using PCR sequencing or gel electrophoresis); or the cell and/or the tissue model do not produce immune rejection or toxicity, and/or the introduced substance does not affect other functions of the cell and/or the tissue model. For example, the detection of repair efficiency may be used as an indicator for evaluating the effectiveness of gene editing, and an exemplary method is shown in Example 4. For example, the off-target efficiency may be detected as an indicator for evaluating the safety of gene editing; for example, the whole genome sequencing may be used to detect the off-target efficiency.

The present application provides use of the gRNA, the one or more isolated nucleic acid molecule(s), the donor nucleic acid molecule, and/or the vector in the manufacture of a medicament for treating a disease, wherein the disease comprises a disease caused by a mutation in the CYP4V2 gene; for example, a disease caused by c.802-8_810del17bpinsGC mutation in the CYP4V2 gene. For example, the disease may include retinitis pigmentosa. For example, the disease may include crystalline retinitis pigmentosa.

The present application provides a pharmaceutical composition comprising the gRNA, the one or more isolated nucleic acid molecule(s), the donor nucleic acid molecule, the vector, and a pharmaceutically acceptable carrier. The carrier should be nontoxic, and should not interfere with the efficacy of the active ingredient.

The pharmaceutical composition described herein may be introduced by a variety of methods, for example, including but not limited to, intravitreal injection (e.g., anterior, medial, or posterior vitreous injection), subconjunctival injection, intracameral injection, bitamporal injection into anterior chamber, intrastromal injection, injection into the subchoroidal space, intracorneal injection, injection into the subretinal space, and locally administration to eyes via intraocular injection. The introduction may comprise the subretinal injection, that is, the injection into the subretinal space, i.e., beneath the neurosensory retina. During the subretinal injection, the injected material (e.g., the targeting vector, the gRNA, and/or the plasmid) is directly introduced between the photoreceptor cells and the retinal pigment epithelium (RPE) layer, and creates a space therebetween.

The present application provides a method for treating the Bietti crystalline dystrophy, comprising the following step: introducing the gRNA (e.g., sgRNA specifically targeting CYP4V2 gene), the one or more isolated nucleic acid molecule(s) (the isolated nucleic acid molecule encoding the sgRNA specifically targeting CYP4V2 gene), the donor nucleic acid molecule (the nucleotide molecule encoding human CYP4V2 gene), and/or the vector into a subject in need thereof. Among them, the introduction enables the subject to obtain a CYP4V2 protein with normal functions.

The method described herein may comprise an *ex vivo* method. In certain instances, the subject-specific induced pluripotent stem cells (iPSCs) may be obtained. Then, the genomic DNA of these iPSC cells may be edited using the method described herein. For example, this method may comprise editing in or near the mutation site of the CYP4V2 gene of the iPSC, such that it does not encode a CYP4V2 protein with mutations. Next, the gene-edited iPSCs may be differentiated into other cells, such as photoreceptor cells or retinal progenitor cells. Finally, the differentiated cells (e.g., photoreceptor cells or retinal progenitor cells) may be implanted into the subj ect.

In certain instances, the subject-specific induced pluripotent stem cells (iPSCs) may be obtained. Then, the induced pluripotent stem cells may be differentiated into any type of cells, such as photoreceptor cells or retinal progenitor cells. In the present application, it may be a 3D retinal organoid. Next, the genomic DNA of these 3D retinal organoid cells may be edited using the method described herein. For example, this method may comprise editing in or near the mutation site of the CYP4V2 gene of the 3D retinal organoid cell, such that it does not encode a CYP4V2 protein with mutations. Finally, the 3D retinal organoid cells may be implanted into the subj ect.

In other instances, the photoreceptor cells or retinal progenitor cells may be isolated from the subject. Next, the genomic DNA of these photoreceptor cells or retinal progenitor cells may be edited using the method described herein. For example, this method may comprise editing in or near the mutation site of the CYP4V2 gene of the photoreceptor cell or retinal progenitor cell, such that it does not have mutated CYP4V2. Finally, the gene-edited photoreceptor cells or retinal progenitor cells may be implanted into the subject.

The method may comprise a comprehensive analysis of the therapeutic agent prior to administration. For example, the entire genome of the corrected cell is sequenced to ensure that no off-target effects, if any, can be at the genomic positions associated with minimal risk to the subject. Moreover, a population of specific cell (including clonal cell population) may be isolated prior to implantation.

The method described herein may comprise the process for cleaving DNA at a precise target position in the genome using a site-directed nuclease, thereby producing single- or double-stranded DNA breakages at the specific position within the genome. Such breakages may be regularly repaired by endogenous cellular processes such as homologous recombination and non-homologous end joining.

The methods described herein may comprise creating one DNA breakage, or two DNA breakages which may be double-stranded breakages or two single-stranded breakages, at the position near the target sequence in the target locus. The breakage may be achieved by a site-directed polypeptide. The site-directed polypeptide (e.g., DNA endonuclease) can introduce double- or single-stranded breakages in the nucleic acid (e.g., genomic DNA). The double-stranded breakage can stimulate the endogenous DNA repair pathway in a cell, such as HR and NHEJ.

Using an exogenous donor template, additional nucleic acid sequences (e.g., the targeting vector) or modifications (e.g., single or multiple base changes or deletions) may be introduced between homologous flanking regions, such that the additional or changed nucleic acid sequences are incorporated into the loci of interest. The exogenous donor may be delivered by a plasmid vector, for example, AAV vector and/or TA cloning vector (e.g., ZT4 vector).

The present application provides a method for regulating the expression of CYP4V2 gene in cells, comprising introducing the gRNA, the one or more isolated nucleic acid molecule(s), and/or the vector into the cells.

The method described herein may involve introducing the gRNA into the cell. For example, the gRNA targets the CYP4V2 gene fragment in the genome of the recipient cell, and cleaves it with the help of nuclease, thereby producing the effects that the chances of the CYP4V2 gene being translated into proteins are reduced and the translated proteins cannot perform a normal function.

The methods described herein may involve introducing the one or more isolated nucleic acid molecule(s) into the cell. For example, the isolated nucleic acid molecule encoding the sgRNA specifically targeting CYP4V2 gene destroys the CYP4V2 gene fragment, thereby producing the effects that the chances of the CYP4V2 gene being translated into proteins are reduced and the translated proteins cannot perform a normal function.

The method described herein may involve introducing the vector into the cell. The vector comprises the isolated nucleic acid molecule and/or the donor nucleic acid molecule. In certain instances, the vector contains the isolated nucleic acid molecule and the donor nucleic acid molecule, such that the CYP4V2 gene in the cell is replaced by the donor nucleic acid molecule, thereby producing the effects that the chances of the CYP4V2 gene being translated into proteins are changed (for example, from the absence of CYP4V2 protein expression to normal expression) and the abnormal function of the translated proteins is changed to a normal function. In certain instances, the vector contains the isolated nucleic acid molecule such that the CYP4V2 gene fragment is destroyed, thereby producing the effects that the chances of the CYP4V2 gene being translated into proteins are reduced and the translated proteins cannot perform a normal function. In other instances, the vector contains the donor nucleic acid molecule, such that the cell contains more CYP4V2 gene fragments, and more CYP4V2 proteins can be transcribed and translated.

Without intention to be limited by any theory, the following Examples are only intended to illustrate the nucleic acid molecules, proteins, preparation methods, uses, etc. in the present application, and are not intended to limit the scope of the claimed invention.

### Examples

### Example 1. Synthesis of sgRNA

### 1. The target of sgRNA

sgRNA was designed for c.802-8_810del17bpinsGCmutation site of CYP4V2 mutant gene. The CRISPR-Cas9 system was used to knock out the CYP4V2 mutant gene in one of the chromosomes from the mutant cell. If the mutations in the two chromosomes were the same, the mutations in the two chromosomes could be knocked out at the same time.

### 2. SgRNA design

The sgRNA was designed with PAM sequence of NNGRRT and NNGRR (*Staphylococcus aureus,* SA; SaCas9) and a length of 21bp in the mutation site region of CYP4V2. The 23 sgRNAs as designed were set forth in SEQ ID NOs: 67-89. The nucleic acid sequences encoding the sgRNAs were shown in Table 2.

**Table 2. Nucleic acid sequence encoding sgRNA**

| | Sense/antisense strand | Sequence | PAM |
|---|---|---|---|
| sgRNA1 | -1 | CATTTCATTGGCCCGTTCGCT (SEQ ID NO: 1) | TTGAA (SEQ ID NO: 24) |
| sgRNA2 | -1 | ATCACATGCTTCTGTTTGGAC (SEQ ID NO: 2) | TTGGA (SEQ ID NO: 25) |
| sgRNA3 | 1 | AGCATGTGATTATCATTCAAA (SEQ ID NO: 3) | GCGAA (SEQ ID NO: 26) |
| sgRNA4 | 1 | TGTGATTATCATTCAAAGCGA (SEQ ID NO: 4) | ACGGG (SEQ ID NO:27) |
| sgRNA5 | 1 | TCATTCAAAGCGAACGGGCCA (SEQ ID NO: 5) | ATGAA (SEQ ID NO: 28) |
| sgRNA6 | 1 | CAAAGCGAACGGGCCAATGAA (SEQ ID NO: 6) | ATGAA (SEQ ID NO: 28) |
| sgRNA7 | 1 | TAGGGTGCATCCAAGTCCAAA (SEQ ID NO: 7) | CAGAA (SEQ ID NO: 29) |
| sgRNA8 | 1 | GGGCCAATGAAATGAACGCCA (SEQ ID NO: 8) | ATGAA (SEQ ID NO: 28) |
| sgRNA9 | 1 | GAAGACTGTAGAGGTGATGGC (SEQ ID NO: 9) | AGGGG (SEQ ID NO: 30) |
| sgRNA10 | -1 | GGCCCTGCGTTTATTTTTGGA (SEQ ID NO: 10) | GGGGG (SEQ ID NO: 31) |
| sgRNA11 | 1 | TGCCCCCTCCAAAAATAAACG (SEQ ID NO: 11) | CAGGG (SEQ ID NO: 32) |
| sgRNA12 | 1 | GAAATAGGCTTAGAAAAATAA (SEQ ID NO: 12) | ATGAA (SEQ ID NO: 28) |
| sgRNA13 | 1 | TAGGCTTAGAAAAATAAATGA (SEQ ID NO: 13) | AAGAA (SEQ ID NO: 33) |
| sgRNA14 | 1 | AAAGAAACTAGCATATTTTAT (SEQ ID NO: 14) | AAGAA (SEQ ID NO: 33) |
| sgRNA15 | 1 | TTTATAAGAAAATGTGTTAAC (SEQ ID NO: 15) | TAGGG (SEQ ID NO: 34) |
| sgRNA16 | -1 | ATGATAATCACATGCTTCTGT (SEQ ID NO: 16) | TTGGA (SEQ ID NO: 25) |
| sgRNA17 | 1 | AATGAACGCCAATGAAGACTG (SEQ ID NO: 17) | TAGAG (SEQ ID NO: 35) |
| sgRNA18 | 1 | TGAAGACTGTAGAGGTGATGG (SEQ ID NO: 18) | CAGGG (SEQ ID NO: 32) |
| sgRNA19 | -1 | TGCGTTTATTTTTGGAGGGGG (SEQ ID NO: 19) | CAGAG (SEQ ID NO: 36) |
| sgRNA20 | -1 | AGGCCCTGCGTTTATTTTTGG (SEQ ID NO: 20) | AGGGG (SEQ ID NO: 30) |
| sgRNA21 | -1 | AAGGCCCTGCGTTTATTTTTG (SEQ ID NO: 21) | GAGGG (SEQ ID NO: 37) |
| sgRNA22 | -1 | GAAAGGCCCTGCGTTTATTTT (SEQ ID NO: 22) | TGGAG (SEQ ID NO: 38) |
| sgRNA23 | -1 | AGAAAGGCCCTGCGTTTATTT (SEQ ID NO: 23) | TTGGA (SEQ ID NO: 25) |

### 3. Detection of sgRNA editing effectiveness in vitro

### (1) In vitro transcription of sgRNA:

1) The primer design for *in vitro* transcription of sgRNA was shown in Table 3.

**Table 3 Primers for in vitro transcription of sgRNA**

| Primer name | Primer sequence |
|---|---|
| F-CYP-sgRNA | TAATACGACTCACTATAGGTTTTAGTACTCTGGAAACAG (SEQ ID NO: 39) |
| R-CYP-sgRNA | ATCTCGCCAACAAGTTGACGAG (SEQ ID NO: 40) |

| | |
|---|---|
| Note: F: Forward (forward strand); R: Reverse (reverse strand) | |

2) Template Construction for *in vitro* transcription of sgRNA
The PCR reaction system was as follows:

| Reagent | Volume (µL) |
|---|---|
| 2×KOD-FX buffer | 25 |
| 2 mM dNTP mix | 10 |
| Sense strand primer | 1.5 |
| Antisense strand primer | 1.5 |
| pX601 plasmid | 0.5 |
| KOD-FX DNA polymerase | 2 |
| Enzyme-free H₂O | ad 50 |

The PCR reaction program was as follows:

| | |
|---|---|
| 94°C | 2 min |
| 98°C | 10 s |
| 58°C | 30 s |
| 68°C | 30 s |
| 68°C | 5 min |

40 cycles
3) 2.0% DNA gel was used for running the gel, and the gel recovery of the *in vitro* transcription template of the gRNA was performed using the OMEGA gel recovery kit in accordance with the following protocols:
a) An equal volume of membrane binding solution was added to the PCR reaction product (1 µL of membrane binding solution per 1 mg gel cut and recovered), heated at 50-60°C for 7 min until all the gels were completely dissolved, mixed by vortexing, and recovered by column.
b) The above liquid was dropped into the recovery column for centrifuging at 10,000×g for 1 min, and the filtrate was discarded.
c) 700 µL of Washing Buffer was added and centrifuged at >13,000×g for 1 min, and the filtrate was discarded.
d) Step c) was repeated.
e) The empty tube was centrifuged at >13,000×g for 10 min.
f) The centrifuge column was transferred to a new 1.5 mL Ep tube and marked. 20-30 µL of Elution Buffer or ddH₂O was added and kept at room temperature for 2 min.
g) After centrifuging at >13,000×g for 1 min, the adsorption column was discarded. The DNA was stored at 2-8°C, with concentration measured and recorded. It was required to be placed at -20°C for long-term storage.

*4) In vitro* transcription of sgRNA (20 µL system):

| Reagent | Volume (µL) |
|---|---|
| 10× Transcription Buffer | 2 |
| rNTP mix | 2 |
| T7 RNA polymerase mix | 2 |
| gRNA gel recovery template | 1 µg (≤14 µL) |
| Diethylpyrocarbonate (DEPC) H₂O | ad 20 |

| | |
|---|---|
| Note: T7 RNA polymerase mix was added at the end. After mixing, the system was placed in a constant temperature incubator at 37°C for reaction; after reaction completion, 2 µL of DNase I was added to react at 37°C for 30 min and then run the gel. | |

5) The gRNA was recovered in accordance with the instructions of the OMEGA gel recovery kit with the same protocols as above.

### (2) Preparation of CYP4V2 sgRNA cleavage template

The DNA of iPSCs derived from patients homozygous for CYP4V2 c.802-8_810del17bpinsGC were extracted, and used as the template to prepare the CYP4V2 sgRNA cleavage template dsDNA.
1) Genomic DNA extraction:
a) The cells were collected by centrifugation at 400×g for 5 min, and the supernatant was discarded. 220 µL of phosphate buffer solution (PBS), 10 mL of RNase Solution, and 20 µL of PK working solution were added to the sample. The cells were resuspended, and left to stand at room temperature for more than 15 min.
b) 250 µL of Buffer GB was added to the cell resuspension, mixed well by vortexing, placed in water bath at 65°C for 15-30 min, and purified by column.
c) 250 µL of absolute ethanol was added to the digestion solution, and mixed well by vortexing for 15-20 s.
d) The gDNA adsorption column was placed in a 2 mL collection tube. The mixture liquid obtained in the above step (including the precipitate) was transferred to the adsorption column, and centrifuged at 12,000×g for 1 min. If the column plugging occurred, the column was centrifuged at 14,000×g for 3-5 min. If the mixture liquid exceeded 750 µL, it was necessary to pass through the column in fractions.
e) The filtrate was discarded, and the adsorption column was placed in the collection tube. 500 µL of Washing Buffer A was added to the adsorption column, and centrifuged at 12,000xg for 1 min.
f) The filtrate was discarded, and the adsorption column was placed in the collection tube. 650 µL of Washing Buffer B was added to the adsorption column, and centrifuged at 12,000xg for 1 min.
g) Step 4 was repeated.
h) The filtrate was discarded, and the adsorption column was placed in the collection tube. The empty tube was centrifuged at 12,000×g for 2 min.
i) The adsorption column was placed in a new 1.5 mL centrifuge tube. 30-100 µL of Elution Buffer preheated to 70°C was added to the center of the membrane of the adsorption column, left to stand at room temperature for 3 min, and then centrifuged at 12,000×g for 1 min.
Note: for DNA-rich tissues, 30-100 µL of Elution Buffer was further added to repeat the elution.
The adsorption column was discarded. The DNA was stored at 2-8°C, with concentration measured and recorded. It was required to be placed at -20°C for long-term storage.
2) The primers used in the PCR process were shown in Table 4:

**Table 4. PCR primers for dsDNA fragment in sgRNA in vitro cleavage experiment**

| Primer name | Primer sequence | PCR product length | Applicable sgRNA |
|---|---|---|---|
| F1-S-CYP4V2-dsDNA | TTAGCGCAATCCAATCTCTAGG (SEQ ID NO: 41) | 449bp | sgRNA1∼12 |
| R1-S-CYP4V2-dsDNA | CTTCATGACTTAGCCTGTTCCCT (SEQ ID NO: 42) | | |
| F2-S-CYP4V2-dsDNA | TTAGCGCAATCCAATCTCTAGG (SEQ ID NO: 41) | 791bp | sgRNA13∼23 |
| R2-S-CYP4V2-dsDNA | CCACATGGAGAACTGAGAGCA (SEQ ID NO: 43) | | |

3) The PCR reaction system was as follows:

| Reagent | Volume (µL) |
|---|---|
| DNA | 0.4 |
| 5× PrimeSTAR buffer | 10 |
| dNTP mix | 4 |
| Sense strand primer | 3 |
| Antisense strand primer | 3 |
| PrimeSTAR HS DNA polymerase | 0.5 |
| DEPC treated water | 29.1 |

The PCR reaction program was as follows:

| | |
|---|---|
| 95°C | 2 min |
| 95°C | 10 s |
| 56°C | 30 s |
| 72°C | 30 s |
| 72°C | 5 min |
| 16°C | 10 min |

30 cycles
4) 1.5 % DNA gel was used for running the gel, and the PCR product was recovered using the OMEGA gel recovery kit in accordance with the same protocols as above:

### (3) In vitro digestion reaction for SaCas9-SgRNA

The reaction system was as follows:

| Reagent | Volume (µL) |
|---|---|
| SaCas9 | 1 |

| | |
|---|---|
| 10× SaCas9 buffer | 2 |
| gRNA (*in vitro* transcription) | 50 ng |
| RHO sgRNA cleavage template | 50 ng |
| DEPC H₂O | ad 20 |

The system was mixed well and reacted at 37°C for 30 min. 3 µL of DNA Loading Buffer was added, mixed, and boiled at 65°C for 5 min. A 2% agarose gel was used to analyze the digestion results.

The theoretical cleavage fragment lengths were shown in Table 5. The experimental results were shown in Figs. 1A-1I. The results showed that sgRNA8, sgRNA12, sgRNA13, sgRNA14, sgRNA16, sgRNA17, and sgRNA18 all had a good cleavage effect on target fragments, and could cleave dsDNA into fragments with corresponding lengths, while other sgRNAs could not cleave dsDNA into corresponding fragments.

**Table 5. Theoretical cleavage lengths for dsDNA fragment in sgRNA in vitro cleavage experiment**

| | dsDNA length | Fragment length after cleavage | |
|---|---|---|---|
| sgRNA1 | 449bp | 154bp | 295bp |
| sgRNA2 | 449bp | 185bp | 264bp |
| sgRNA3 | 449bp | 147bp | 302bp |
| sgRNA4 | 449bp | 143bp | 306bp |
| sgRNA5 | 449bp | 135bp | 314bp |
| sgRNA6 | 449bp | 150bp | 299bp |
| sgRNA7 | 449bp | 172bp | 277bp |
| sgRNA8 | 449bp | 120bp | 329bp |
| sgRNA9 | 449bp | 96bp | 353bp |
| sgRNA10 | 449bp | 76bp | 373bp |
| sgRNA11 | 449bp | 68bp | 381bp |
| sgRNA12 | 791bp | 216bp | 575bp |
| sgRNA13 | 791bp | 220bp | 571bp |
| sgRNA14 | 791bp | 240bp | 551bp |
| sgRNA15 | 791bp | 256bp | 535bp |
| sgRNA16 | 791bp | 282bp | 509bp |
| sgRNA17 | 791bp | 339bp | 452bp |
| sgRNA18 | 791bp | 351bp | 440bp |
| sgRNA19 | 791bp | 368bp | 423bp |
| sgRNA20 | 791bp | 374bp | 417bp |
| sgRNA21 | 791bp | 375bp | 416bp |
| sgRNA22 | 791bp | 377bp | 414bp |
| sgRNA23 | 791bp | 378bp | 413bp |

### Example 2. Validation of sgRNA target at the cellular level

### 1. sgRNA synthesis

According to *in vitro* cleavage experiment, effective sgRNAs were selected: sgRNA8; sgRNA12; sgRNA13; sgRNA14; sgRNA16; sgRNA17; and sgRNA18. According to the sequence of digestion site Bbs1, the Bbs1 digestion sites were added in the upstream and downstream of the designed sgRNA (in capital letters). The nucleic acid molecule encoding the sgRNA was shown in Table 6:

**Table 6. Nucleic acid sequence encoding sgRNA**

| No. | Sequence |
|---|---|
| CYP4V2-sgRNA8-F | ACACGgggccaatgaaatgaacgccaG (SEQ ID NO: 44) |
| CYP4V2-sgRNA8-R | AAAACtggcgttcatttcattggcccC (SEQ ID NO: 45) |
| CYP4V2-sgRNA12-F | ACACGgaaataggcttagaaaaataaG (SEQ ID NO: 46) |
| CYP4V2-sgRNA12-R | AAAACttatttttctaagcctatttcC (SEQ ID NO: 47) |
| CYP4V2-sgRNA13-F | ACACGtaggcttagaaaaataaatgaG (SEQ ID NO: 48) |
| CYP4V2-sgRNA13-R | AAAACtcatttatttttctaagcctaC (SEQ ID NO: 49) |
| CYP4V2-sgRNA14-F | ACACGaaagaaactagcatattttatG (SEQ ID NO: 50) |
| CYP4V2-sgRNA14-R | AAAACataaaatatgctagtttctttC (SEQ ID NO: 51) |
| CYP4V2-sgRNA16-F | ACACGAtgataatcacatgcttctgtG (SEQ ID NO: 52) |
| CYP4V2-sgRNA16-R | AAAACatgataatcacatgcttctgtC (SEQ ID NO: 53) |
| CYP4V2-sgRNA17-F | ACACGaatgaacgccaatgaagactgG (SEQ ID NO: 54) |
| CYP4V2-sgRNA17-R | AAAACcagtcttcattggcgttcattC (SEQ ID NO: 55) |
| CYP4V2-sgRNA18-F | ACACGtgaagactgtagaggtgatggG (SEQ ID NO: 56) |
| CYP4V2-sgRNA18-R | AAAACccatcacctctacagtcttcaC (SEQ ID NO: 57) |

### 2. SgRNA vector construction and plasmid extraction

### (1) SgRNA annealing

Each sgRNA (F, R) synthesized as above was diluted to 50 µmol, respectively. 5 µL of each sgRNA (F, R) was used to prepare sgRNA mixs 1-7.

T4 PNK and 10X T4 Ligation Buffer were thawed on ice for later use. The following reaction system was prepared:

| Reagent | Volume (µL) |
|---|---|
| sgRNA mix | 7 |
| 10× T4 Ligation Buffer | 1 |
| T4 PNK | 2 |
| Total volume | 10 |

The reaction system prepared in step 5 was placed in a PCR instrument, and the following reaction program was run:

| | |
|---|---|
| 37 °C | 30 min |
| 95 °C | 5 min |

| Ramp down to 25 °C at 5 °C /min | |
|---|---|
| 25 °C | Forever |

The reaction product was recovered.

### (2) Vector digestion

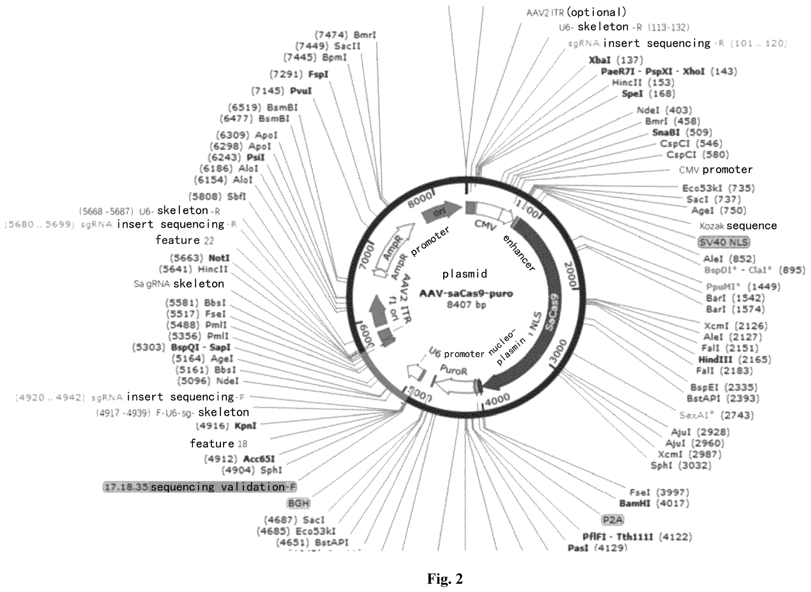

For the construction of sgRNA vector, the plasmid used was AAV-saCas9-puro vector. The plasmid map was shown in Fig. 2.

BbsI digestion was used to release the binding site for sgRNA. The following digestion reaction system was prepared in a 1.5 mL PCR tube:

| | |
|---|---|
| Plasmid | 15 µg |
| 2× Cutsmart buffer | 30 µL |
| Enzyme | 12 µL |
| H₂O | ad 258 µL |
| Total volume | 300 µL |

After digestion for 1-2 h (or digestion at 4 °C overnight), the system was recovered and purified. The concentration was determined, and then it was diluted to 50 ng/µL.

### (3) Ligation

The recovered vector from the previous step and the annealed sgRNA were used to prepare the following ligation system (200 µL PCR tube):

| Reagent | Volume (µL) |
|---|---|
| Digested vector | 1 |
| Annealed sgRNA | 1 |
| 2× T4 Ligation Buffer | 5 |
| T4 Ligase | 1 |
| H₂O | 3 |
| Total volume | 11 |

The ligation reaction system from the previous step was placed at 37°C for about 1-2 hours to complete the sgRNA vector construction.

### (4) Plasmid transformation

1) TransStbl3 competent cells (TransGen Biotech, CD-521-02) were placed on ice for thawing.
2) 1 µL of ligation product was added to 50 µL of competent cells, and incubated on ice for half an hour, subjected to heat shock at 42°C for 90 s, and kept on ice for 2 min.
3) 500 µL of antibiotic-free medium was added at 37°C and shaken at 200 rpm for 1 h.
4) The system was centrifuged at 800 rpm/min for 5 min, and the supernatant was discarded for remaining about 100 µL. The LB agar medium containing ampicillin was used to screen positive clones.
5) On the next day, the positive colony was picked (500 µL of medium) and shaken for culturation for 3-4 hours, 200 µL of which was sent for sequencing.

### (5) Plasmid extraction (in accordance with Omega Endotoxin-Free Plasmid Maxiprep Kit)

1) The plasmids confirmed correct by sequencing were shaken for culturation overnight (50-200 mL), and incubated on a shaker at 37°C for 12-16 h to amplify the plasmid, and extracted on the next day (shaking for less than 16 h).
2) 50-200 mL of bacteria was centrifuged at 4,000×g for 10 min at room temperature, to collect the bacterial cells.
3) The medium was discarded. 10 mL of Solution I/RNase mixture was added to the pellet, and the cells were completely resuspended by pipetting or vortexing.
4) 10 mL of Solution II was added. The centrifuge tube was capped and gently inverted for 8-10 times to obtain a clear lysate.
5) 5 mL of pre-chilled N3 Buffer was added. The centrifuge tube was capped and gently inverted for 10 times until a white flocculent precipitate was formed. The system may be left to stand and incubated at room temperature for 2 min.
6) A syringe filter was prepared. The plunger in the syringe was pulled out. Then the syringe was vertically placed on a suitable test-tube support, and a collection tube was placed at the outlet of the lower end of the syringe, with the opening of the syringe faced upward. The lysate was immediately poured into the syringe of the filter. The cell lysate was remained in the syringe for 5 min. At this time, white floccules would float on the surface of the lysate. The cell lysate may have flowed out of the filter syringe port. The cell lysate was collected in a new 50 mL tube. The plunger of the syringe was carefully and gently inserted into the syringe, and pushed slowly such that the lysate flowed into the collection tube.
7) One-tenth volume of ETR Solution (blue) was added to the filtered lysate that had flowed out. The tube was inverted for 10 times, and then left to stand in an ice bath for 10 minutes.
8) The above lysate was kept in water bath at 42°C for 5 min. The lysate would be cloudy again. At this time, the lysate was centrifuged at 4,000×g for 5 minutes at 25°C. The ETR buffer would form a blue layer at the bottom of the tube.
9) The supernatant was transferred to another new 50 mL tube, and a half volume of absolute ethanol at room temperature was added. The tube was gently inverted for 6-7 times, and placed at room temperature for 1-2 min.
10) A HiBind^{®} DNA Maxi binding column was cased in a 50 mL collection tube, and 20 mL of filtrate was added to the HiBind^{®} DNA Maxi binding column, for centrifuging at 4,000×g for 3 min at room temperature. The filtrate was discarded.
11) The HiBind^{®} DNA Maxi binding column was cased in the same collection tube to repeat step 10 until all the remaining filtrate was bound to the HiBind^{®} DNA Maxi binding column, for centrifuging under the same conditions.
12) The HiBind^{®} DNA Maxi binding column was cased in the same collection tube, and 10 mL of HBC Buffer was added to the HiBind^{®} DNA Maxi binding column, for centrifuging at 4,000×g for 3 min at room temperature. The filtrate was discarded.
13) The HiBind^{®} DNA Maxi binding column was cased in the same collection tube, and 15 mL of DNA Wash Buffer (diluted with absolute ethanol) was added to the HiBind^{®} DNA Maxi binding column, for centrifuging at 4,000×g for 3 min at room temperature. The filtrate was discarded.
14) The HiBind^{®} DNA Maxi binding column was cased in the same collection tube, and 10 mL of DNA Wash Buffer (diluted with absolute ethanol) was added to the HiBind^{®} DNA Maxi binding column, for centrifuging at 4,000×g for 3 min at room temperature. The filtrate was discarded.
15) The matrix of the HiBind^{®} DNA Maxi binding column (empty) was dried by centrifuging in an empty state at maximum speed (not exceeding 6,000×g) for 10 minutes.
16) (Optional) The HiBind^{®} DNA Maxi binding column was further air-dried. Any of the following processes was (optionally) chosen to further dry the HiBind^{®} DNA Maxi binding column before the elution of DNA (if necessary):
   a) The HiBind^{®} DNA Maxi binding column was placed in a vacuum container for 15 min to dry the ethanol: the column was moved to the vacuum chamber at room temperature and all vacuum chamber devices were connected. The vacuum chamber was sealed and vacuumed for 15 min. The HiBind^{®} DNA Maxi binding column was removed for the next operation. b) The column was dried in a vacuum oven or at 65°C for 10-15 min. The HiBind^{®} DNA Maxi binding column was removed for the next operation.
17) The HiBind^{®} DNA Maxi binding column was placed in a clean 50 mL centrifuge tube, 1-3 mL of Endotoxin-free Elution Buffer was directly added onto the HiBind^{®} DNA Maxi binding column matrix (the amount added depending on the expected final product concentration), and left to stand at room temperature for 5 min.
18) The DNA was eluted by centrifuging at 4,000×g for 5 min.
19) The column was discarded, and the DNA product was stored at -20°C.
20) The extracted plasmids were sequenced again to ensure that the constructed plasmids were correct.

### 3. Validation of sgRNA effectiveness in 293T cells

### (1) 293T cell culture

1) Thawing of frozen cells
   a) The temperature of the thermostatic water bath was adjusted to 37°C. The frozen cells were taken out from the liquid nitrogen. By clamping the lid with tweezers, the container was shaken quickly in the water.
   b) The liquid was transferred to a 10 mL centrifuge tube, 10 mL of culture medium was slowly added, and shaken gently to homogenize the liquid. After tightening the lid, the tube was centrifuged at 1,000 rpm/min for 3 min.
   c) Removing the supernatant and adding an appropriate amount of medium. The cell at the bottom was pipetted gently, and then the cells were transferred to a culture flask for culturing in an incubator. 293T cells were cultured at 37°C with 5% CO₂, using high-glucose DMEM supplemented with 10% fetal bovine serum and 100 U/ml penicillin/streptomycin as the medium.
2) Cell passage
   a) The morphology and density of the cells were observed under an inverted microscope. When the cells in the culture flask reached 80%-90% confluence, the cells were passaged.
   b) The old medium in the cell culture flask was washed out, and the flask was washed with PBS for 3 times. 500 µl of EDTA-containing trypsin was added to the culture flask, and incubated for about 1 min in the incubator. When the intercellular space became larger and the cells became round, 1 mL of medium was immediately added to the culture flask to stop the digestion. The cells were pipetted gently, and after all the cells floated from the bottom of the flask, the liquid in the culture flask was transferred to a centrifuge tube and centrifuged at 1,000 rpm/min for 2 min.
   c) The supernatant was discarded, and 2 mL of medium was added to the centrifuge tube to resuspend the cells. The cell suspension was dispensed into 4 new culture flasks, and 4 mL of medium was added to each flask. The culture flask was shaken gently such that the cells were mixed evenly and plated onto the culture flask, and then placed into a cell incubator for culturing.
   d) On the day before transfection, 293T cells (1 million) having a density of 80%, under cell stretching, and with uniform intercellular space were added to each well, and subjected to plasmid transfection when the cells reached 80-90% confluence on the next day.

### (2) 293T cell transfection by polyethyleneimine (PEI) method

1) 1.5 mL EP tubes were numbered in the above order. 250 µL of DMEM medium (serum-free) was added to each tube, and 1.5 µg of AAV-CYP4V2- sgRNA8, sgRNA 12, sgRNA 13, sgRNA 14, sgRNA 16, sgRNA 17, sgRNA 18 plasmids and empty plasmids were added, respectively. After mixing well by vortexing, 7.5 µL of PEI transfection reagent was added to each tube, mixed well by vortexing, and left to stand at room temperature for 20 min before transfection. A negative control was also set.
2) The system was dropwise added to the culture medium (the culture medium in 6-well plate plus serum-free DMEM = 2 mL/well), cultured in an incubator, and half of the medium was changed within 12-18 hours. On the next day, the GFP expression was observed under a fluorescence microscope to assess the transfection efficiency, and the transfected plasmid was continued to be cultured if the transfection efficiency was good.
3) Screening of resistant cells
   a) Solution formulation: 10 mg/mL (mother solution) puromycin was diluted to 1 µg/mL puromycin for cell screening.
   b) Medium changing: two days after transfection, 3 mL of 293T cell medium containing puromycin was added to each well (including the negative control group) to screen for positively transfected cells. Then the survival state of the cells was observed every day. The culture medium was refreshed every 2 days, and a corresponding amount of puromycin was added when changing the medium. At the time that the cells in the negative control well were completely dead while the experimental group and the control group had viable cells (indicating a successful transfection), the antibiotic screening was terminated and the culture medium was changed to the normal medium.

### (3) Extraction of genomic DNA from 293T cells

1) After the cells in the 6-well plate reached 80-90% confluence, the cells were passaged to a 6 cm culture dish.
2) After the cells reached 80-90% confluence, the cells were harvested for extracting the genomic DNA. The whole process took about 7-10 days. The genomic DNA was extracted using the cell extraction kit from Vazyme Biotech, and the experimental protocols were as follows:
   a) The cells were collected by centrifugation at 400×g for 5 min, and the supernatant was discarded. 220 µL of PBS, 10 mL of RNase Solution, and 20 µL of PK working solution were added to the sample. The cells were resuspended, and left to stand at room temperature for more than 15 min.
   b) 250 µL of Buffer GB was added to the cell resuspension, mixed well by vortexing, placed in water bath at 65°C for 15-30 min, and purified by column.
   c) 250 µL of absolute ethanol was added to the digestion solution, and mixed well by vortexing for 15-20 s.
   d) The gDNA adsorption column was placed in a 2 mL collection tube. The mixture liquid obtained in the above step (including the precipitate) was transferred to the adsorption column, and centrifuged at 12,000×g for 1 min. If the column plugging occurred, the column was centrifuged at 14,000×g for 3-5 min. If the mixture liquid exceeded 750 µL, it was necessary to pass through the column in fractions.
   e) The filtrate was discarded, and the adsorption column was placed in the collection tube. 500 µL of Washing Buffer A was added to the adsorption column, and centrifuged at 12,000xg for 1 min.
   f) The filtrate was discarded, and the adsorption column was placed in the collection tube. 650 µL of Washing Buffer B was added to the adsorption column, and centrifuged at 12,000xg for 1 min.
   g) Step 4 was repeated.
   h) The filtrate was discarded, and the adsorption column was placed in the collection tube. The empty tube was centrifuged at 12,000×g for 2 min.
   i) The adsorption column was placed in a new 1.5 mL centrifuge tube. 30-100 µL of Elution Buffer preheated to 70°C was added to the center of the membrane of the adsorption column, left to stand at room temperature for 3 min, and then centrifuged at 12,000×g for 1 min.
      Note: for DNA-rich tissues, 30-100 µL of Elution Buffer was further added to repeat the elution.
   j) The adsorption column was discarded. The DNA was stored at 2-8°C, with concentration measured and recorded. It was required to be placed at -20°C for long-term storage.

### (4) T7E1 digestion experiment

1) Using the genomic DNA extracted from 293T cells transfected with AAV-CYP4V2-sgRNA8, sgRNA12, sgRNA13, sgRNA14, sgRNA16, sgRNA17, sgRNA18 plasmids as above, around the target site, the extracted genomic DNA was amplified using the upstream or downstream primers, for PCR of DNA fragments (the primer sequences were shown in Table 7).

**Table 7. Primers for T7E1 digestion experiment**

| Primer name | Sequence | PCR product length |
|---|---|---|
| T7E1-F | CTCATTAGCGCAATCCAATCTC (SEQ ID NO: 58) | 793bp |
| T7E1-R | AGCAATCTCTGGCTGCTCTT (SEQ ID NO: 59) | |

2) The liquid recovery kit (OMEGA Gel Extraction Kit (200) D2500-02) was used to recover the liquid DNA from the above PCR products, and the DNA recovery protocols were as follows:
a) An equal volume of membrane binding solution was added to the PCR reaction product (1 µL of membrane binding solution per 1 mg gel cut and recovered), heated at 50-60°C for 7 min until all the gels were completely dissolved, mixed by vortexing, and recovered by column;
b) The above liquid was dropped into the recovery column for centrifuging at 10,000×g for 1 min, and the filtrate was discarded;
c) 700 µL of Washing Buffer was added and centrifuged at >13,000×g for 1 min, and the filtrate was discarded;
d) Step c) was repeated;
e) The empty tube was centrifuged at >13,000×g for 10 min;
f) The centrifuge column was transferred to a new 1.5 mL Ep tube and marked. 20-30 µL of Elution Buffer or ddH₂O was added and kept at room temperature for 2 min;
g) After centrifuging at >13,000×g for 1 min, the adsorption column was discarded. The DNA was stored at 2-8°C, with concentration measured and recorded. It was required to be placed at -20°C for long-term storage.

3) Validation of sgRNA efficiency by T7E1 digestion experiment

The product obtained by PCR recovery or gel cutting recovery as above was subjected to the T7E1 digestion reaction.
a) Annealing system for T7E1 digestion (19.5 µL):

| Reagent | Volume (µL) |
|---|---|
| NEB Buffer 2 | 2 |
| PCR or gel cutting recovery product | X (500 ng or 1,000 ng) |
| Deionized H₂O | ad 19.5 |

b) Annealing program for T7E1 digestion:

| | | |
|---|---|---|
| 95 °C | 2 min | |
| 95°C | To 85°C | Temperature decreased at 2°C/s |
| 85°C | To 25°C | Temperature decreased at 0.1°C/s |
| 16°C | ∞ | |

c) Reaction system for T7E1 digestion

| Reagent | Volume (µL) |
|---|---|
| Annealing product | 9.75 or 9.5 |
| T7E1 enzyme | 0.25 or 0.5 |

The system was incubated at 37 °C for 20 min.
d) Gel electrophoresis of digestion product
Gel formulation: 2.5% gel, with double dye added.
Gel electrophoresis program: 140 V, 20 min to 30 min.

e) The gel electrophoresis results were checked.

The digestion results were shown in Fig. 3; the fragment lengths for each cleaved fragment were shown in Table 8.

**Table 8. Fragment length table after sgRNA cleavage**

| Template | PCR fragment length | Fragment length after digestion | |
|---|---|---|---|
| CYP4V2-sgRNA8 | 793bp | 348bp | 445bp |
| CYP4V2-sgRNA13 | | 224bp | 569bp |
| CYP4V2-sgRNA17 | | 358bp | 435bp |
| CYP4V2-sgRNA18 | | 370bp | 423bp |

The results showed that sgRNA8, sgRNA13, sgRNA17, and sgRNA18 all had a cleavage effect on the target, and the cleaved fragments were consistent with those shown in Table 8.

### (5) Gene sequencing for PCR fragments containing cleavage sites of genomic DNA (nested peak test)

The amplified DNA fragments as above were sequenced, and the sequencing maps were shown in Figs. 4A-4C. The results showed that sgRNA13, sgRNA17, and sgRNA18 had high cleavage efficiencies, and nested peak appeared near the sgRNA cleavage site.

### 4. Validation of double sgRNA cleavage efficiency

### (1) Construction and extraction of double sgRNA-AAV-saCas9-puro plasmid

According to the above experimental results, the cleavage efficiencies of sgRNA13.17.18 were high. In the AAV-saCas9-sgRNA13 plasmid constructed in the above experiments, the U6 promoter-sgRNA17-saScaffold fragment and the U6 promoter-sgRNA18-sa-gRNA Scaffold were respectively inserted after the NotI digestion between sa-gRNA Scaffold and AAV ITR region to construct AAV-saCas9-sgRNA13-sgRNA17 and AAV-saCas9-sgRNA13-sgRNA18 plasmids.
1) The PCR primer sequences for U6 promoter-sgRNA17-saScaffold fragment and U6 promoter-sgRNA18-sa-gRNA Scaffold fragment were shown in Table 9.

**Table 9. U6-SACFFOLD primer sequence**

| | |
|---|---|
| Primer name | Sequence |
| U6-SACFFOLD-F | CTTGTTGGCGAGATTTTTGCGGCC gagggcctatttcccatgattc (SEQ ID NO:60) |
| U6-SACFFOLD-R | CCATCACTAGGGGTTCCTGC (SEQ ID NO:61) |

Among them, the capital letters were the homologous arm sequence.
The PCR reaction system was as follows:

| Reagent | Volume (µL) |
|---|---|
| 2×KOD-FX buffer | 25 |
| 2 mM dNTP mix | 10 |
| Sense strand primer | 1.5 |
| Antisense strand primer | 1.5 |
| AAV-saCas9-puro plasmid | 0.5 |
| KOD-FX DNA polymerase | 2 |
| Enzyme-free H₂O | ad 50 |

The PCR reaction program was as follows:

| | |
|---|---|
| 94°C | 2 min |
| 98°C | 10 s |
| 58°C | 30 s |
| 68°C | 30 s |
| 68°C | 5 min |

40 cycles
2) 2.0% DNA gel was used for running the gel, and the gel recovery of the *in vitro* transcription template of the gRNA was performed using the OMEGA gel recovery kit in accordance with the following protocols:
a) An equal volume of membrane binding solution was added to the PCR reaction product (1 µL of membrane binding solution per 1 mg gel cut and recovered), heated at 50-60°C for 7 min until all the gels were completely dissolved, mixed by vortexing, and recovered by column;
b) The above liquid was dropped into the recovery column for centrifuging at 10,000×g for 1 min, and the filtrate was discarded;
c) 700 µL of Washing Buffer was added and centrifuged at >13,000×g for 1 min, and the filtrate was discarded;
d) Step c) was repeated;
e) The empty tube was centrifuged at >13,000×g for 10 min;
f) The centrifuge column was transferred to a new 1.5 mL Ep tube and marked. 20-30 µL of Elution Buffer or ddH₂O was added and kept at room temperature for 2 min;
g) After centrifuging at >13,000×g for 1 min, the adsorption column was discarded. The DNA was stored at 2-8°C, with concentration measured and recorded. It was required to be placed at -20°C for long-term storage.

3) Vector digestion
NotI digestion release site was used. The following digestion reaction system was prepared in a 1.5 mL PCR tube:

| | |
|---|---|
| Plasmid | 15 µg |
| 2× Cutsmart buffer | 30 µL |
| Enzyme | 12 µL |
| H₂O | ad 258 µL |
| Total volume | 300 µL |

After digestion for 1-2 h/(K digestion overnight), the system was recovered and purified. The concentration was determined, and then it was diluted to 50 ng/µL.
4) Ligation
The recovered vector from the previous step and the annealed sgRNA were used to prepare the following ligation system (200 µL PCR tube):

| Reagent | Volume (µL) |
|---|---|
| Digested vector | 1 |
| Annealed sgRNA | 1 |
| 2× T4 Ligation Buffer | 5 |
| T4 Ligase | 1 |
| H₂O | 3 |
| Total volume | 11 |

The ligation reaction system from the previous step was placed at 37°C for about 1-2 hours to complete the sgRNA vector construction.
5) Plasmid transformation
a) TransStbl3 competent cells were placed on ice for thawing.
b) 1 µL of ligation product was added to 50 µL of competent cells, and incubated on ice for half an hour, subjected to heat shock at 42°C for 90 s, and kept on ice for 2 min.
c) 500 µL of antibiotic-free medium was added at 37°C and shaken for culturation at 200 rpm for 1 h.
d) The system was centrifuged at 800 rpm/min for 5 min, and the supernatant was discarded for remaining about 100 µL. The LB agar medium containing ampicillin was used to screen positive clones.
e) On the next day, the positive colony was picked (500 µL of medium) and shaken for culturation for 3-4 hours, 200 µL of which was sent for sequencing.

6) The protocols for plasmid extraction (in accordance with Omega Endotoxin-free Plasmid Maxiprep Kit) was identical to the previous protocols.

### (2) Validation of the effectiveness of double sgRNA and single sgRNA in 293T cells

1) 293T cell culture
a) Thawing of frozen cells
   i) The temperature of the thermostatic water bath was adjusted to 37°C. The frozen cells were taken out from the liquid nitrogen. By clamping the lid with tweezers, the container was shaken quickly in the water.
   ii) The liquid was transferred to a 15 mL centrifuge tube, 10 mL of culture medium was slowly added, and shaken gently to homogenize the liquid. After tightening the lid, the tube was centrifuged at 1,000 rpm/min for 3 min.
   iii) Removing the supernatant and adding an appropriate amount of medium. The cell at the bottom was pipetted gently, and then the cells were transferred to a culture flask for culturing in an incubator. 293T cells were cultured at 37°C with 5% CO₂, using high-glucose DMEM supplemented with 10% fetal bovine serum and 100 U/ml penicillin/streptomycin as the medium.
b) Cell passage
   i) The morphology and density of the cells were observed under an inverted microscope. When the cells in the culture flask reached 80%-90% confluence, the cells were passaged.
   ii) The old medium in the cell culture flask was washed out, and the flask was washed with PBS for 3 times. 500 µL of EDTA-containing trypsin was added to the culture flask, and incubated for about 1 min in the incubator. When the intercellular space became larger and the cells became round, 1 mL of medium was immediately added to the culture flask to stop the digestion. The cells were pipetted gently, and after all the cells floated from the bottom of the flask, the liquid in the culture flask was transferred to a centrifuge tube and centrifuged at 1,000 rpm/min for 2 min.
   iii) The supernatant was discarded, and 2 mL of medium was added to the centrifuge tube to resuspend the cells. The cell suspension was dispensed into 4 new culture flasks, and 4 mL of medium was added to each flask. The culture flask was shaken gently such that the cells were mixed evenly and plated onto the culture flask, and then placed into a cell incubator for culturing.
   iv) On the day before transfection, 293T cells (1 million) having a density of 80%, under cell stretching, and with uniform intercellular space were added to each well, and subjected to plasmid transfection when the cells reached 80-90% confluence on the next day.

(2) 293T cell transfection by polyethyleneimine (PEI) method
a) 1.5 mL EP tubes were numbered in the above order. 250 mL of DMEM medium (serum-free) was added to each tube, and 1.5 mg of AAV-CYP4V2-sgRNA 13 plasmid; AAV-CYP4V2-sgRNA 17 plasmid; AAV-CYP4V2-sgRNA 18 plasmid; AAV-CYP4V2-sgRNA 13-sgRNA 17 plasmid; AAV-CYP4V2-sgRNA 13-sgRNA 18 plasmid as well as empty plasmid were successively added. After mixing well by vortexing, 7.5 mL of PEI transfection reagent was added to each tube, mixed well by vortexing, and left to stand at room temperature for 20 min before transfection. A negative control was also set.
b) The system was dropwise added to the culture medium (the culture medium in 6-well plate plus serum-free DMEM = 2 mL/well), cultured in an incubator, and half of the medium was refreshed within 12-18 hours. On the next day, the GFP expression was observed under a fluorescence microscope to assess the transfection efficiency, and the transfected plasmid was continued to be cultured if the transfection efficiency was good.
c) Screening of resistant cells
   i) Solution formulation: 10 mg/mL (mother solution) puromycin was diluted to 1 µg/mL puromycin for cell screening.
   ii) Medium changing: two days after transfection, 3 mL of 293T cell medium containing puromycin was added to each well (including the negative control group) to screen for positively transfected cells. Then the survival state of the cells was observed every day. The culture medium was refreshed every 2 days, and a corresponding amount of puromycin was added when refreshing the medium. At the time that the cells in the negative control well were completely dead while the experimental group and the control group had viable cells (indicating a successful transfection), the antibiotic screening was terminated and the culture medium was changed to the normal medium.

3) Extraction of genomic DNA from 293T cells
a) After the cells in the 6-well plate reached 80-90% confluence, the cells were passaged to a 6 cm culture dish.
b) After the cells reached 80-90% confluence, the cells were harvested for extracting the genomic DNA. The whole process took about 7-10 days. The genomic DNA was extracted using the cell extraction kit from Vazyme Biotech, and the experimental protocols were as follows:
   i) The cells were collected by centrifugation at 400×g for 5 min, and the supernatant was discarded. 220 µL of PBS, 10 mL of RNase Solution, and 20 µL of PK working solution were added to the sample. The cells were resuspended, and left to stand at room temperature for more than 15 min.
   ii) 250 µL of Buffer GB was added to the cell resuspension, mixed well by vortexing, placed in water bath at 65°C for 15-30 min, and purified by column.
   iii) 250 µL of absolute ethanol was added to the digestion solution, and mixed well by vortexing for 15-20 s.
   iv) The gDNA adsorption column was placed in a 2 mL collection tube. The mixture liquid obtained in the above step (including the precipitate) was transferred to the adsorption column, and centrifuged at 12,000×g for 1 min. If the column plugging occurred, the column was centrifuged at 14,000×g for 3-5 min. If the mixture liquid exceeded 750 µL, it was necessary to pass through the column in fractions.
   v) The filtrate was discarded, and the adsorption column was placed in the collection tube. 500 µL of Washing Buffer A was added to the adsorption column, and centrifuged at 12,000xg for 1 min.
   vi) The filtrate was discarded, and the adsorption column was placed in the collection tube. 650 µL of Washing Buffer B was added to the adsorption column, and centrifuged at 12,000×g for 1 min.
   vii) Step 4 was repeated.
   viii) The filtrate was discarded, and the adsorption column was placed in the collection tube. The empty tube was centrifuged at 12000×g for 2 min.
   ix) The adsorption column was placed in a new 1.5 mL centrifuge tube. 30-100 µL of Elution Buffer preheated to 70°C was added to the center of the membrane of the adsorption column, left to stand at room temperature for 3 min, and then centrifuged at 12,000×g for 1 min.
      Note: for DNA-rich tissues, 30-100 µL of Elution Buffer was further added to repeat the elution.
   x) The adsorption column was discarded. The DNA was stored at 2-8°C, with concentration measured and recorded. It was required to be placed at -20°C for long-term storage.

4) Gene sequencing for PCR fragments containing cleavage sites of genomic DNA (nested peak test)
a) The primers were designed for the above DNA, across the target cleavage site, to amplify the fragment. The primer sequences were shown in Table 10.

**Table 10. Amplification primers containing the cleavage site sequence of the genomic DNA**

| Primer name | Sequence | PCR product length |
|---|---|---|
| F | AGAGATTTTAATTAAGGTACctcattagcgcaatccaatctc (SEQ ID NO: 62) | 833 bp |
| R | GAAACATGTATTAATACGCGagcaatctctggctgctctt (SEQ ID NO: 63) | |

The portion in capital letters was the homologous arm sequence (the homologous arm after the digestion of PMD-19T-MCS vector with MluI and KpnI).
b) Vector linearization and ligation
The plasmid vector was PMD-19T-MCS vector, and the protocols for vector linearization were the same as above, wherein the enzymes used were MluI enzyme and KpnI enzyme. The PCR product was recovered and purified, sequenced, and ligated to the PMD-19T-MCS linearized vector. The protocols were the same as above.
c) Plasmid transformation
i) TransStbl3 competent cells were placed on ice for thawing.
ii) 1 µL of ligation product was added to 50 µL of competent cells, and incubated on ice for half an hour, subjected to heat shock at 42°C for 90 s, and kept on ice for 2 min.
iii) 500 µL of antibiotic-free medium was added at 37°C and shaken at 200 rpm for 1 h.
iv) The system was centrifuged at 800 rpm/min for 5 min, and the supernatant was discarded for remaining about 100 µL. The LB agar medium containing ampicillin was used to screen positive clones.
v) On the next day, the positive colonies were picked, 80 of which were sent for sequencing.

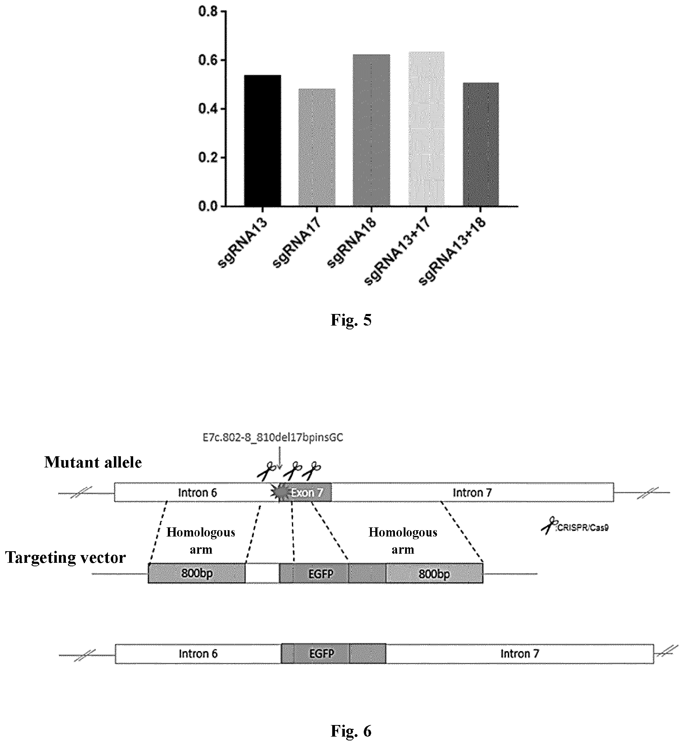

d) Analysis of positive percentage
After calculating the sequencing map, the positive percentage was as follows:
The positive percentage for sgRNA13 was 54%, the positive percentage for sgRNA17 was 48.5%, the positive percentage for sgRNA18 was 62.5%, the positive percentage for sgRNA13+17 was 64%, and the positive percentage for sgRNA13+18 was 51.2%.
It could be found that to a certain extent, the positive percentage for double sgRNA increased.
The experimental results were shown in Fig. 5.

### Example 3. Screening of donor (donor nucleic acid molecule)

### 1. Donor design

The HDR method was adopted in the donor design. Considering that the cleavage site for sgRNA13 was in Intron 6 and the cleavage sites for sgRNA17 and sgRNA18 were in Exon 7, the EGFP gene was inserted between the cleavage sites for sgRNA17 and sgRNA18 (specifically Exon7: c.802+42 site), and 800 bp homologous arms were added to both sides of the EGFP gene.

The donor design map was shown in Fig. 6.

The donor sequence was set forth in any of SEQ ID NOs: 64-66.

### 2. Construction and extraction of PMD 19-T-donor plasmid

The protocols were the same as above.

### 3. Validation of donor effectiveness in 293T cells

### (1) 293T cell culture

The protocols were the same as above.

### (2) 293T cell co-transfection by polyethyleneimine (PEI) method

1) 1.5 mL EP tubes were numbered in the above order. 250 µL of DMEM medium (serum-free) was added to each tube, and 1.5 µg of AAV-CYP4V2-sgRNA13 plasmid; AAV-CYP4V2-sgRNA17 plasmid; AAV-CYP4V2-sgRNA18 plasmid; AAV-CYP4V2-sgRNA13-sgRNA17 plasmid; AAV-CYP4V2-sgRNA13-sgRNA18 plasmid as well as empty plasmid were successively added. After mixing well by vortexing, 1.5 µg of corresponding PMD19-T-donor plasmid for each tube was added (the corresponding relationship was shown below), and mixed well. 7.5 µL of PEI transfection reagent was added to each tube, mixed well by vortexing, and left to stand at room temperature for 20 min before transfection. A negative control was also set.

**Table 11. Correspondence Table for sgRNA and donor**

| sgRNA | Corresponding donor |
|---|---|
| AAV-CYP4V2-sgRNA13 | PMD19-T-dono13 |
| AAV-CYP4V2-sgRNA17 | PMD19-T-donol17 |
| AAV-CYP4V2-sgRNA18 | PMD19-T-donol18 |
| AAV-CYP4V2-sgRNA13-sgRNA17 | PMD19-T-dono13 |
| | PMD19-T-dono17 |
| AAV-CYP4V2-sgRNA13-sgRNA18 | PMD19-T-dono13 |
| | PMD19-T-dono18 |

2) The system was dropwise added to the culture medium (the culture medium in 6-well plate plus serum-free DMEM = 2 mL/well), cultured in an incubator, and half of the medium was refreshed within 12-18 hours. On the next day, the GFP expression was observed under a fluorescence microscope to assess the transfection efficiency, and the transfected plasmid was continued to be cultured if the transfection efficiency was good.
3) Screening of resistant cells
a) Solution formulation: 10 mg/mL (mother solution) puromycin was diluted to 1 µg/mL puromycin for cell screening.
b) Medium changing: two days after transfection, 3 mL of 293T cell medium containing puromycin was added to each well (including the negative control group) to screen for positively transfected cells. Then the survival state of the cells was observed every day. The culture medium was refreshed every 2 days, and a corresponding amount of puromycin was added when changing the medium. At the time that the cells in the negative control well were completely dead while the experimental group and the control group had viable cells (indicating a successful transfection), the antibiotic screening was terminated and the culture medium was changed to the normal medium.

4) Flow cytometry sorting
The cells after antibiotic screening (about 7 days) were subjected to flow cytometric sorting, and the protocols were as follows:
a) The medium in the 6-well plate was aspirated and the cells were washed twice with DuLbecco's phosphate buffer (DPBS);
b) 500 µL of 0.05% trypsin was added for incubating and digesting at 37°C for 4 min;
c) 3-5 volumes of DMEM were added to neutralize the trypsin, and centrifuged at 800 r/min for 2 min;
d) The supernatant was aspirated, and PBS was added to resuspend the cells, and centrifuged at 800 r/min for 2 min; the process was repeated once;
e) The supernatant was aspirated, and 200 µL of PBS containing 2% FBS was added to resuspend the cells;
f) The obtained liquid in the above step was added to the filter tube, such that all the liquid passes through the filter screen;
g) The sample was placed in the instrument to run. The positive cells after screening were transferred to a 12-well or 6 well plate for culturing.

5) Extraction of genomic DNA from 293T cells
a) After the cells in the 6-well plate reached 80-90% confluence, the cells were passaged to a 6 cm culture dish.
b) After the cells reached 80-90% confluence, the cells were harvested for extracting the genomic DNA. The whole process took about 7-10 days. The genome was extracted using the cell extraction kit from Vazyme Biotech, and the experimental protocols were as above.

6) Sequencing of target fragment
a) The primers were designed around the upstream and downstream of the donor, and the digestion sites were added upstream and downstream of the primers.
b) Vector linearization and ligation
   The plasmid vector was PMD-19T-MCS vector, and the protocols for vector linearization were the same as above, wherein the enzymes used were MluI enzyme and KpnI enzyme. The PCR product was recovered and purified, sequenced, and ligated to the PMD-19T-MCS linearized vector. The protocols were the same as above.
c) Plasmid transformation
   The protocols were the same as above.
d) Sequencing
   Each plasmid was picked and sent for sequencing, and the sequencing results were used in statistics.

### Example 4. In vitro validation of sgRNA gene editing efficiency using 3D retinal tissue

### 1. Extraction and culture of renal epithelial cells from patients

Using the renal epithelial cell isolation and culture kit provided by Beijing Cellapy, the experimental protocols were as follows:
1) The UrinEasy isolation complete medium, supplements, gelatin, and washing solution were brought to the cell room.
2) The 12-well plate, 50 mL centrifuge tube, 15 mL centrifuge tube, electric pipettor, pipette, sucker, 5 mL pipettor and tips, and 1 mL pipettor and tips were irradiated under UV lamp; and a water bath kettle at 37°C was turned on.
3) Urine collection: with gloves and disinfection, the urine (preferably midstream urine) was collected, and sealed with the sealing film.
4) 750 µL/well of gelatin was added to coat the bottom of the dish (3 wells) for not less than half an hour, and kept at 37°C.
5) The outer surface of the urine bottle was disinfected with 75% alcohol, and the urine was dispensed into a 50 mL conical-bottom centrifuge tube. The tube was sealed and centrifuged at 400×g for 10 min.
6) The UrinEasy isolation complete medium + supplements were removed and formulated (5 mL of basic medium per 0.5 mL of supplements).
7) Along the upper surface, the supernatant was slowly aspirated by pipette to 1 mL with the minimum speed.
8) The liquid was resuspended in a 15 mL centrifuge tube, 10 mL of washing solution was added and mixed well, then centrifuged at 200×g for 10 min.
9) The gelatin was aspirated from the 12-well plate, and the plate was washed once with the washing solution (500 µL). 750 µL of UrinEasy isolation complete medium was added to each well and kept at 37°C.
10) The 15 mL centrifuge tube was taken out, and 0.2 mL of cell pellet remained.
11) The cell pellet was resuspended with UrinEasy isolation complete medium: one well for male and two wells for female, denoted as day 0 (D0).
12) Observation:
   D1: whether there was contamination was observed;
   D2: the isolation medium was supplemented - female: 500 µL/well; male: 250 µL/well;
   D4: If the cells were not adhered: half volume of the medium was refreshed every two days by slowly adding 1 mL of isolation complete medium along the wall.
13) Until the adherence occurred: after the cells appeared to adhere (3-7 days or 9~10 days), UrinEasy expansion complete medium was selected for culturing
   (500 µL for refreshing the whole medium). At approximately 9-12D (not exceeding 14D) after adherence, the cells at 80-90% confluence were passaged to 6-well plate, 6 cm dish, and 10cm dish, respectively, and then frozen for later use.

### (2) Induction of iPSCs

The patient-derived (c.802-8_810del17bpinsGC) renal epithelial cells were induced into iPSCs in accordance with the following protocols:
1) The somatic cells were digested and passaged when the cells reached 70-90% confluence. The cells were seeded in a 96-well plate with a controlled density of 5,000 to 15,000 cells/well. 3 density gradients might be set according to the cell conditions, with 3 duplicate wells for each gradient. The day of cell seeding was recorded as day -1.
2) Day 0: The confluence and state of cells were observed under the microscope, and duplicate wells with different gradients were selected for digestion and counting. The wells with 10,000-20,000 cells were chosen for reprogramming. The formulation for Reprogramming Medium A was as follows:

| Reprogramming Medium A | Volume |
|---|---|
| Somatic Cell Culture Medium | 10 mL |
| Reprogramming Supplement I | 10 µL |

3) The Reprogramming Supplement II was first centrifuged, and then 97 µL of Reprogramming Medium A was added to the tube of Reprogramming Supplement II, and mixed well to prepare the Reprogramming Medium B. 100 µL of Reprogramming Medium B was added to a chosen eligible 96-well, and the plate was placed back into the incubator.
4) Days 1-2: The cells were observed under the microscope and photographed to record the changes in cell morphology. If the cell morphology was obviously changed, the Reprogramming Medium B was removed and replaced with the Reprogramming Medium A for further culturing. If the morphological changes were not obvious, the medium might not be changed.
5) Day 3: If the cell morphology had subjected to obvious deformation in the first two days, and the cell growth speed was relatively fast, the cells were digested by trypsin and passaged. Depending upon the cell state and amount, the cells were transferred to 2-6 wells of a 6-well plate, and the Reprogramming Medium C was added to form the single-cell adherence as much as possible. The formulation for Reprogramming Medium C was as follows:

| Reprogramming Medium C | Volume |
|---|---|
| Reprogramming Medium A | 9.8 mL (remaining from above) |
| Reprogramming Supplement III | 5 µL |

6) Day 4: The adherence of the cells was observed. If most of the cells adhered well, the medium was replaced with fresh somatic cell culture medium for further culturing.
7) Day 5: The cells were observed under the microscope. If a small clone cluster (clone pellet with more than 4 cells) was formed, the somatic cell culture medium was replaced with Reproeasy Human Somatic Cell Reprogramming Medium. If no small clone cluster was formed, the cells were further observed for one or two days before replacing the medium with Reproeasy Human Somatic Cell Reprogramming Medium.
8) Days 6-8: The cells were observed under the microscope. If the small clone cluster became larger and there were more than 10 cells in a clone pellet, Reproeasy Human Somatic Cell Reprogramming Medium was directly replaced with PSCeasy Human Pluripotent Stem Cell Medium (or PGM1 Human Pluripotent Stem Cell Medium). If a relatively large number of dead cells were observed before changing the medium, the cells might be washed with PBS equilibrated at room temperature before changing the medium.
9) Days 9-20: The cells were observed under the microscope and photographed to record the changes in cell morphology. The fresh PSCeasy Human Pluripotent Stem Cell Medium equilibrated at room temperature was replaced daily.
10) Day 21: The cells were observed under the microscope. If a single cell clone could fill the entire 10x field of view, a 1 mL syringe needle (or other equipment such as glass needles) was used to cut and pick the clone into a 48-cell plate coated with Matrigel in advance (if the clone was in good condition with massive cells and fast growth, it could be directly picked into a 24-well plate).
11) The clone was picked out and seeded with PSCeasy Human Pluripotent Stem Cell Resuscitation Medium. After the cells adhered to the wall, the medium was replaced with PSCeasy Human Pluripotent Stem Cell Medium for further culturing to the desired passage.

### (3) Induction of patient-derived 3D retinal cells

The specific protocols were shown in Table 12.

**Table 12. Protocols for induction of patient-derived 3D retinal cells**

| | | | |
|---|---|---|---|
| Stage 1 | D0 | The iPSCs were seeded in a T25 flask at a density of 30,000/cm² and cultured until the cell density reached about 70%. | E8 medium |
| Stage 2 | D1 | The cells were digested with Dispase (2 mg/mL), and 3:1 E8:NIM+rock inhibitor (Y27632) medium was added to suspend the cultured cells in a 10 cm dish. (The embryoid body (EB) was formed on the next day and existed alone.) | E8 + NIM |
| | D2 | The medium was changed (E8:NIM=1:1). | |
| | D3 | The medium was changed (E8:NIM=1:3). | |
| | D4-5 | The medium was changed (Complete NIM). | NIM |
| | D6 | The medium was changed (NIM + 50 ng/mL BMP4). | |
| | D7 | EBs with good morphology (single-chamber, clear) were chosen, and 1 mL (about 200 EBs) medium was dropwise added to the adherent 6-well plate, and then (1 mL NIM+250 µL FBS)/well was added. | |
| | D8 | The medium was changed ((4 mL NIM + 25 ng/mL BMP4)/well). | |
| | D9-15 | Half of the medium was refreshed every other day (NIM medium). | |
| Stage 3 | D16 | The aggregated cell pellets were transferred into a 15 mL centrifuge tube with a 10 µL pipette tip, such that they sunk to the bottom naturally. The supernatant was removed, and 5 mL RDM was added to resuspend. The suspension was seeded into a 6 cm dish, and the medium was refreshed every 2-3 days. | RDM |
| | D18-20 | By observation under 4× microscope, the retinal neurospheres (pellets) with bright outer rings on the surface were collected gently and slowly, and transferred to a 15 mL centrifuge tube containing 5 mL RDM. The collected retinal neurospheres (pellets) were transferred into a 6 cm dish. | |
| | D20-30 | The medium was refreshed every 2-3 days. The retinal organoids were continually chosen based on the presence or absence of bright outer ring in the appearance. During this period, some organoids might be gradually transformed towards non-retinal neurospheres. After 30 days, the remaining retinal neurospheres could maintain their morphology ever since. | |

### (4) Construction and coating of AAV8 virus

1) Plasmid amplification: the constructed AAV vector (both the sgRNA and the donor were engineered from the plasmid pX601-AAV-CMV-SaCas9 (Addgene Plasmid #61591); for the convenience of description, hereinafter, the plasmid for sgRNA was called as AAV-CYP4V2-sgRNA, and the plasmid for the donor was called as AAV-CYP4V2-donor, wherein AAV-CYP4V2-donor contained partial EGFP sequence, same as for PMD-19T-donor sequence as above), packaging plasmid, and helper plasmid should be subjected to the endotoxin-free maxiprep extraction. The Qiagen maxiprep extraction kit was used for the maxiprep plasmid extraction in accordance with the same protocols as above.
2) AAV8-293T cell transfection: the cell density was observed on the day of transfection, and at 80-90% confluence, the vector plasmid, packaging plasmid, and helper plasmid were used for transfection.
3) AAV8 virus collection: the virus particles were present in both packaging cells and culture supernatant. Both the cells and the culture supernatant could be collected for a good yield.
4) After AAV purification, it was stored at -80°C for long-term storage.

### (5) In vitro validation of sgRNA gene editing efficiency using 3D retinal tissue

### 1) Use of AAV8 in infecting 3D retinal tissue.

### 2) The gene editing efficiency was verified.

### 1) T7E1 digestion experiment

The protocols were the same as above.

### 2) TOPO PCR cloning

In order to quantify the editing efficiencies of AAV-CYP4V2-sgRNA and AAV-CYP4V2-donor as well as statistically analyze the cleavage efficiencies of the experimental and control groups, Zero Blunt TOPO PCR Cloning Kit from Invitrogen was used for experiments. The clones were picked and sent for Sanger sequencing.
a) The DNA obtained after infecting the 3D retinal tissue with AAV virus was used as the amplification template, and the DNA polymerase from Platinum SuperFi^{™} series of Invitrogen was used for PCR reactions. The reaction system was as follows (50 µL system):

| | |
|---|---|
| 5× SuperFi^{™} Buffer | 10 µL |
| 10 mM dNTP mix | 1 µL |
| Sense primer | 1.25 µL |
| Antisense primer | 1.25 µL |
| gDNA | 50 ng |
| Platinum^{™} SuperFi^{™} DNA polymerase | 0.5 µL |
| DEPC H₂O | ad 50 µL |

b) Touch down PCR program:
The protocols were the same as above.
c) TOPO PCR cloning reaction was as follows:

| Reagent | Volume (µL) |
|---|---|
| Fresh PCR product | 0.5-4 |
| Salt solution | 1 |
| ddH₂O | To 5 |
| pCR^{™}-Blunt II-TOPO^{®} | 1 |
| Total volume | 6 |

The above system was prepared, mixed gently, and left to stand at room temperature for 5 min;
Then the system was placed on ice for the transformation into competent cells.

d) TOPO PCR cloning reaction for the transformation into competent cells
2 µL of the above TOPO PCR cloning reaction liquid was added to 50 or 100 µL of competent cells, and placed on ice for 5-30 min;
The system was subjected to heat shock at 42°C for 30 sec without shaking;
It was immediately transferred on ice, and 250 µL of S.O.C. medium was added;
The system was shaken at 200 rpm at 37°C for 1 h for thawing;
A corresponding amount of LB medium (25 µg/mL Zeocin (bleomycin) added) and 100 µL of the above bacteria solution were used for plating, and cultured in an incubator at 37°C overnight.

e) The clones were picked and sent for Sanger sequencing.
The next morning, the bacteria plates were taken out, and 80 clones per plate were picked, shaken at 200 rpm at 37°C for 3-4 h; 200 µL for each clone sample was sent for Sanger sequencing.

### Example 5. Validation of gene editing efficiency in a humanized mouse model

### 1. Validation of gene editing efficiency in a humanized mouse model

### 1) Construction of humanized mice

The construction of humanized mice was completed by Beijing Biocytogen Co., Ltd.

For the human mutation site, the Beijing Biocytogen Gene Biotechnology Co., Ltd. was commissioned to construct the humanized mouse models for CYP4V2.

### 2) Technical contents:

a) Design and construction of the sgRNA that recognizes the target sequence;
b) Construction of the CRISPR/Cas9 vector for the cleavage of target gene;
c) Activity detection of sgRNA/Cas9;
d) Design and construction of the targeting vector for gene knock-in with the replacement of exons 6-8 (including intron sequence) in accordance with the design scheme;
   *In vitro* transcription of sgRNA/Cas9 mRNA;
e) Injection of sgRNA/Cas9 mRNA and targeting vector into mouse fertilized eggs;
f) Detection and propagation of F0 generation mice with CYP4V2 gene knock-in;
g) Acquisition and genotype identification of F1 generation heterozygous mice with CYP4V2 gene knock-in (southern blotting DNA hybridization validation, including one exogenous probe and one endogenous probe).

### 3) Technical method:

a) Amplification and sequencing of the target sequence in mouse genome, design and construction of CRISPR/Cas9 vector plasmid for the target sequence, and activity detection:
b) Extraction of mouse genomic DNA and amplification of the sgRNA recognizing the target sequence;
c) Design and construction of the CRISPR/Cas9 vector plasmid for the cleavage of target sequence;
d) Activity detection of sgRNA/Cas9 using the detection kit independently developed by Biocytogen;
e) Design and construction of the targeting vector for CYP4V2 gene knock-in by selecting highly active sgRNA/Cas9 target site sequence information;
f) Pronucleus injection of sgRNA/Cas9 mRNA and targeting vector into mouse fertilized eggs, and acquisition of F0/F1 generation positive mice. This experimental process mainly included the following:
   *In vitro* transcription of sgRNA/Cas9 mRNA;
   Collection of mouse fertilized eggs;
   Injection of mouse fertilized eggs with RNA and targeting vector into the mouse fertilized eggs;
   Transplantation of the fertilized eggs into the fallopian tubes of surrogate mice;
   Genotype identification and propagation of F0 generation mice with humanized point mutation of CYP4V2 gene;
   Acquisition and genotype identification of F1 generation mice with humanized point mutation of CYP4V2 gene (including PCR detection and southern blotting hybridization validation).

### (2) Feeding and breeding

After obtaining the two kinds of humanized mice, the F1 generation heterozygous mice were inbred to obtain a sufficient number of F2 or F3 generation humanized homozygous mice as soon as possible for AAV virus injection.

### (3) Injection of AAV virus into the subretinal space in mice

The AAV-CYP4V2-sgRNA (providing SaCas9) and AAV-CYP4V2-donor vectors were packaged into adeno-associated virus and injected into the retina of CYP4V2 mutant model mice to verify the editing and repair efficiency of the designed sgRNA and donor *in vivo.* Taking AAV-CYP4V2-sgRNA13+AAV-CYP4V2-donor13 as example, the detailed protocols were as follows (the experimental protocols for sgRNA17-18 group were the same).

### 1) Experimental design

### (1) Blank control group: physiological saline.

### (2) Experimental group: AAV-CYP4V2-sgRNA 13 (providing SaCas9) and AAV-CYP4V2-donor13.

### 2) Selection of adeno-associated virus (AAV) serotype

The AAV2/8 serotype with a better preference for the retina was selected.

### 3) Virus packaging

The AAV-CYP4V2-donor13 and AAV-CYP4V2-sgRNA13 vectors were packaged with AAV2/8 and AAV-helper into adeno-associated virus (AAV).

### 4) Injection experiment in mice

20 CYP4V2 mutant model mice were used in the experiment and divided into 4 groups (5 in each group).

The experimental protocols were as follows:
a) Blank control group: the mice were injected with 2 µL of physiological saline in each eye.
b) Experimental group: the packaged AAV-CYP4V2-sgRNA 13 and AAV-CYP4V2-donor13 viruses were mixed in equal amounts at a ratio of 1:1, and the mice were injected with 2 µL (1E10vg) in each eye.
c) The therapeutic effect was detected after one month.

The experimental protocols were as follows:
a) The pupils were dilated with 1% atropine at 30 min before injection, and dilated again before anesthetization.
b) The mice were anesthetized by intraperitoneal injection at a ratio of 80 mg/kg ketamine and 8 mg/kg xylazine. Then the mice were placed in front of the animal experiment platform of the ophthalmic surgery microscope, and a drop of 0.5% proparacaine was dropped on the eyes of mice for local anesthesia. The fluorescein sodium stock solution was added to the AAV virus at a concentration of 100:1, and mixed by low-speed centrifugation.
c) A minipore was pricked by insulin needle in advance in the ciliary pars plana of the mouse eyeball, through which a microsyringe needle passed to enter the vitreous chamber of the mouse eyeball. At this time, an appropriate amount of 2% hydroxymethyl cellulose was dropped on the mouse eyeball such that the mouse fundus could be seen under the microscope. Then the needle was inserted into the contralateral periphery subretinal space while keeping off the lens. The AAV virus with fluorescein sodium was slowly pushed-in, with an injection volume of 1 µl in each eye. The fluorescein sodium served as the indicator for judging whether it was injected into the subretinal space.
d) Whether the mouse was normal or not was observed after injection, and the neomycin eye ointment was applied to prevent infection.

### 5) Evaluation of the effect of gene editing therapy:

The experimental results found that the therapeutic effects of each experimental group were essentially the same, so the AAV-CYP4V2-sgRNA13 and AAV-CYP4V2-donor13 virus group was selected as a representative to illustrate the therapeutic effect of gene editing.

### (4) Editing efficiency assessment of AAV8- AAV-CYP4V2-sgRNA in humanized mice

The validation protocols referred to the section "(5) *In vitro* validation of sgRNA gene editing efficiency using 3D retinal tissue" in Example 4.

The experimental results found that the therapeutic effects of each experimental group were essentially the same, so the AAV-CYP4V2-sgRNA13 and AAV-CYP4V2-donor13 virus group was selected as a representative to illustrate the therapeutic effect of gene editing.
The functional changes of mice after treatment were evaluated by ERG (retinal electrophysiology). After the treatment, the retinal function of the mutant mouse model was improved;
After the sections were taken, the morphological changes of the mouse retina were observed by HE staining, and the morphology was close to the condition of the retina of wild-type mouse;
Whether the gene editing therapeutic vector was expressed in the corresponding site of the retina was observed by immunofluorescence staining, and the gene editing therapeutic vector was expressed in the corresponding site of the retina;
The changes of retinal tissue morphology were observed through a series of staining labels for special retinal markers, and it was found that the retinal tissue morphology of mice was improved after treatment.
Finally, it should be noted that: the above examples are only used to illustrate the technical solutions of the present application, and are not limitative; although the present application has been described in detail with reference to the above examples, those of ordinary skill in the art should understand that the technical solutions described in the above examples can be modified, or some technical features can be replaced equivalently; and these modifications or replacements do not make the essence of the corresponding technical solutions departing from the spirit and scope of the technical solutions of the examples of the present application.

## Claims

1. A gRNA specifically targeting polypeptide 2, subfamily V, family 4, cytochrome P450 (CYP4V2) gene, which specifically binds to the nucleotide sequence within 200 bp upstream or downstream of c.802-8_810del17bpinsGC mutation site.

2. The gRNA according to claim 1, comprising a nucleotide sequence set forth in any of SEQ ID NOs: 74, 78-80, and 82-84.

3. The gRNA according to any of claims 1-2, comprising 5'-(X)n-SEQ ID NO: 74, 78-80, 82-84-skeleton sequence-3', wherein X is a base selected from any of A, U, C and G, and n is any integer from 0 to 15.

4. The gRNA according to any of claims 1-3, wherein the gRNA is a single-stranded guide RNA (sgRNA).

5. One or more isolated nucleic acid molecule(s) encoding the gRNA specifically targeting CYP4V2 gene according to any of claims 1-4.

6. A donor nucleic acid molecule comprising a normal wild-type nucleotide sequence within 800 bp upstream or downstream of c.802-8_810del17bpinsGC mutation site of CYP4V2 gene.

7. The nucleic acid molecule according to claim 6, comprising a nucleotide sequence set forth in any of SEQ ID NOs: 64-66.

8. A vector comprising the isolated nucleic acid molecule according to claim 5 and/or the donor nucleic acid molecule according to any of claims 6-7.

9. The vector according to claim 8, wherein the isolated nucleic acid molecule and the donor nucleic acid molecule are in a same vector.

10. The vector according to any of claims 8-9, which is a viral vector.

11. A cell comprising the isolated nucleic acid molecule according to claim 5, the donor nucleic acid molecule according to any of claims 6-7, and/or the vector according to any of claims 8-10.

12. The cell according to claim 11, comprising HEK293 cells, kidney epithelial cells, and/or induced pluripotent stem cells.

13. The cell according to any of claims 11-12, which has been modified to have a differentiation potential.

14. The cell according to any of claims 11-13, which can be differentiated into a 3D-retinal organoid.

15. A pharmaceutical composition comprising the gRNA according to any of claims 1-4, the one or more isolated nucleic acid molecule(s) according to claim 5, the donor nucleic acid molecule according to any of claims 6-7, and/or the vector according to any of claims 8-10, and a pharmaceutically acceptable carrier.

16. Use of the gRNA according to any of claims 1-4, the one or more isolated nucleic acid molecule(s) according to claim 5, the donor nucleic acid molecule according to any of claims 6-7, and/or the vector according to any of claims 8-10 in the manufacture of a medicament for treating a disease, wherein the disease includes diseases caused by c.802-8_810del17bpinsGC mutations in CYP4V2 gene.

17. The use according to claim 16, wherein the disease includes Bietti crystalline dystrophy.

18. A method for treating Bietti crystalline dystrophy, comprising the following step: introducing the gRNA according to any of claims 1-4, the one or more isolated nucleic acid molecule(s) according to claim 5, the donor nucleic acid molecule according to any of claims 6-7, and/or the vector according to any of claims 8-10 into a subject in need thereof.

19. The method according to any of claim 18, wherein the introducing results in a CYP4V2 protein with a normal function.

20. The method according to any of claims 18-19, wherein the introducing comprises injection.

21. The method according to any of claims 18-20, wherein the introducing comprises injection in the subretinal space.

22. A method of regulating the CYP4V2 gene expression in a cell, comprising introducing into the cell the gRNA according to any of claims 1-4, the one or more isolated nucleic acid molecule(s) according to claim 5, and/or the vector according to any of claims 8-10.
